# EUROPEAN PATENT APPLICATION

(11) **EP 1 978 028 A1**
(43) Date of publication of application: **08.10.2008**
(21) Application number: 07007202.0
(22) Date of filing: 05.04.2007
(51) Int. Cl.: C07K 14/47, C12N 9/12, C12N 9/16, C12Q 1/68

(54) **Use of gene variants of the human MEIS1, BTBD9, MAP2K5, LBXCOR1, PTPRD or A2BP1 gene for diagnostic and therapeutic approaches to restless legs syndrome (RLS)**

(71) Applicant: Max-Planck-Gesellschaft zur Förderung der Wissenschaften e.V., 80539 München (DE)
(72) Inventor: Winkelmann, Juliane, 80804 München (DE); Müller-Myhsok, Bertram, 81241 München (DE)
(74) Representative: Vossius & Partner

(57) **Abstract**

The present invention relates to an oligo- or polynucleotide which can be used in the diagnosis and therapy of restless legs syndrome (RLS). Furthermore, the invention relates to methods of diagnosing a predisposition for or RLS and a method for selecting a therapy to prevent and treat RLS. Moreover, a method for determining the dose of a drug used for treating a patient suffering from RLS and a method to test the efficacy of a drug used for treating RLS in treating other dopaminergic diseases, sleep disorders or spinocerebellar ataxias as well as the use of a drug effective in treating RLS for testing the efficacy of said drug in treating other dopaminergic diseases, sleep disorders or spinocerebellar ataxias. The invention also envisages methods of identifying an inhibitor, pro-drug or drug capable of modulating the activity of a peptide or protein of the invention, a diagnostic kit for detection of a single nucleotide polymorphism and gene therapies on the basis of gene variants detected with the present invention.

SEQUENCE LISTING

<110>  Max-Planck-Gesellschaft zur Förderung der Wissenschaften
       e.V.

<120>  USE OF GENE VARIANTS OF THE HUMAN MEIS1, BTBD9, MAP2K5 OR LBXCOR1
       GENE FOR DIAGNOSTIC AND THERAPEUTIC APPROACHES TO RESTLESS LEGS
       SYNDROME (RLS)

<130>  N1641 EP

<160>  40

<170>  PatentIn version 3.3

<210>  1
<211>  201
<212>  DNA
<213>  homo sapiens

<400>  1
tgctgcttga gagtgtctgt tagctgggct tcaatccacg tccctgtaga ttttaatgtc      60

cctacagact gctggcagcc tgcttccagc tgtggcaggc atgatgcagt gaattgcttt     120

tgaatgttct ttactcaaat aggcagtaat tacaagctta aagaggttta gtggtccagt     180

aaactacata atcacccctg a                                               201


<210>  2
<211>  201
<212>  DNA
<213>  homo sapiens

<400>  2
tcaggggtga ttatgtagtt tactggacca ctaaacctct ttaagcttgt aattactgcc      60

tatttgagta aagaacattc aaaagcaatt cactgcatca cgcctgccac agctggaagc     120

aggctgccag cagtctgtag ggacattaaa atctacaggg acgtggattg aagcccagct     180

aacagacact ctcaagcagc a                                               201


<210>  3
<211>  201
<212>  DNA
<213>  homo sapiens

<400>  3
caatgaaacc agtgacaacg gaggttgact ttgaacatgg tatgcaggtc agcacaactt      60

gttgcaaatc ctaattaagc cagtcttctt gttttcagtg tgtctgtaag tatctggtca     120

gagaaatgca aacaacaggc tgtgggtcat gcaacccgat ggggcaggac ttgactccca     180

tttccctgga gagggcatag g                                               201


<210>  4
<211>  201
<212>  DNA
<213>  homo sapiens

<400> 4
cctatgccct ctccagggaa atgggagtca agtcctgccc catcgggttg catgacccac 60

agcctgttgt ttgcatttct ctgaccagat acttacagac ccactgaaaa caagaagact 120

ggcttaatta ggatttgcaa caagttgtgc tgacctgcat accatgttca aagtcaacct 180

ccgttgtcac tggtttcatt g 201

<210> 5
<211> 201
<212> DNA
<213> homo sapiens

<400> 5
aaatccttta atcttacttt ttagtgtgtc taacaggata aaatggaaaa tactaacata 60

tcatacactg gaaaaaaact aaaccaacta gattgacgaa taagacaagg ccctgtagat 120

cttgagaaag agagatcttc tctgttctga gacttctgga gatcctcaaa atcattcagc 180

caggtattag ttttgtgtaa a 201

<210> 6
<211> 201
<212> DNA
<213> homo sapiens

<400> 6
tttacacaaa actaatacct ggctgaatga ttttgaggat ctccagaagt ctcagaacag 60

agaagatctc tctttctcaa gatctacagg gccttgtctt gttcgtcaat ctagttggtt 120

tagttttttt ccagtgtatg atatgttagt attttccatt ttatcctgtt agacacacta 180

aaaagtaaga ttaaaggatt t 201

<210> 7
<211> 201
<212> DNA
<213> homo sapiens

<400> 7
atgtagccat atttaaggct attgctttca ttctggaggt tcttgcaaat tgactctggg 60

ttctgcatat tctgagggga ggaggcaggg ccaacattgt tgacaagtct cccttttgct 120

tgctgccttt ccacaaagct agcagagcca gaagagaact ggccatagaa acaactgaaa 180

ccacaatagt aggtcaattt t 201

<210> 8
<211> 201
<212> DNA
<213> homo sapiens

<400> 8
aaaattgacc tactattgtg gtttcagttg tttctatggc cagttctctt ctggctctgc 60

tagctttgtg gaaaggcagc aagcaaaagg gagacttgtc aacaatgttg gccctgcctc 120

ctcccctcag aatatgcaga acccagagtc aatttgcaag aacctccaga atgaaagcaa 180

tagccttaaa tatggctaca t 201

<210> 9
<211> 201
<212> DNA
<213> homo sapiens

<400> 9
tataatagag tagaaggaat ggagcaggag aaactgcatg ggcacagagt gggcagatca 60

tgaaaggctc tgcattttaa gctgtggatc ttggacttta cgccataggt gaagaggagc 120

cctgagaagt tttagtagag gagtgacatg acttcgttcg atttttacaa ctttcatcaa 180

gatacatgtt cctaactaca a 201

<210> 10
<211> 201
<212> DNA
<213> homo sapiens

<400> 10
ttgtagttag gaacatgtat cttgatgaaa gttgtaaaaa tcgaacgaag tcatgtcact 60

cctctactaa aacttctcag ggctcctctt cacctatggc ataaagtcca agatccacag 120

cttaaaatgc agagcctttc atgatctgcc cactctgtgc ccatgcagtt tctcctgctc 180

cattccttct actctattat a 201

<210> 11
<211> 201
<212> DNA
<213> homo sapiens

<400> 11
actgcatggg cacagagtgg gcagatcatg aaaggctctg cattttaagc tgtggatctt 60

ggactttatg ccataggtga agaggagccc tgagaagttt cagtagagga gtgacatgac 120

ttcgttcgat ttttacaact ttcatcaaga tacatgttcc taactacaag gagccccacc 180

tacttggttc tgacatatca c 201

<210> 12
<211> 201
<212> DNA
<213> homo sapiens

<400> 12
gtgatatgtc agaaccaagt aggtggggct ccttgtagtt aggaacatgt atcttgatga 60

aagttgtaaa aatcgaacga agtcatgtca ctcctctact gaaacttctc agggctcctc 120

ttcacctatg gcataaagtc caagatccac agcttaaaat gcagagcctt tcatgatctg 180

cccactctgt gcccatgcag t 201

<210> 13
<211> 201
<212> DNA
<213> homo sapiens

<400> 13
tctcttccaa acagaacaag aagtcttcag ggattcagta atagagatga cagaatgcca 60

tgtcttcaag tgaaaataaa atgatagctt tctctcttct gtttatccat ctgcaattca 120

gtaggacaaa tgcaatactc tttttaaaaa atgggctctg gaacctcaat aaaaagaagt 180

gagttctctg attcataaat a 201

<210> 14
<211> 201
<212> DNA
<213> homo sapiens

<400> 14
tatttatgaa tcagagaact cacttctttt tattgaggtt ccagagccca ttttttaaaa 60

agagtattgc atttgtccta ctgaattgca gatggataaa cagaagagag aaagctatca 120

ttttattttc acttgaagac atggcattct gtcatctcta ttactgaatc cctgaagact 180

tcttgttctg tttggaagag a 201

<210> 15
<211> 201
<212> DNA
<213> homo sapiens

<400> 15
aaaagcagta aatatgtaaa gagaaaattt gtcaactgat attggaagat ctgacctagg 60

tcacacataa atcttccaga caagagctgc agggcaagcc ataatttcag agagtaaaag 120

agaaaatgaa agctatagct ggtgtcttct ctaagttaca taggtagatt cattttagtt 180

ttctgattgt gcctctgaat a 201

<210> 16
<211> 201
<212> DNA
<213> homo sapiens

<400> 16
tattcagagg cacaatcaga aaactaaaat gaatctacct atgtaactta gagaagacac 60

cagctatagc tttcattttc tcttttactc tctgaaatta tggcttgccc tgcagctctt 120

gtctggaaga tttatgtgtg acctaggtca gatcttccaa tatcagttga caaattttct 180

ctttacatat ttactgcttt t 201

<210> 17

<211>  201
<212>  DNA
<213>  homo sapiens

<400>  17
attgtcccaa agtactagtt agacatagaa aacactcaac acacgcaaaa tgtataagca      60

ggaatgaggg cttggacaag ttagttataa acagcatttc atatgttgtg aggtgcaggc     120

agtacatcag ccctaacaat attattgcgt tcctctgaac tctgctctca cagctaagca     180

tttggaaggc agacccagaa t                                               201


<210>  18
<211>  201
<212>  DNA
<213>  homo sapiens

<400>  18
attctgggtc tgccttccaa atgcttagct gtgagagcag agttcagagg aacgcaataa      60

tattgttagg gctgatgtac tgcctgcacc tcacaacata tgaaatgctg tttataacta     120

acttgtccaa gccctcattc ctgcttatac attttgcgtg tgttgagtgt tttctatgtc     180

taactagtac tttgggacaa t                                               201


<210>  19
<211>  201
<212>  DNA
<213>  homo sapiens

<400>  19
aaaagttgac ctgaccagtg tatgaaattc aaacactgta taggaacaag gtaaaagcac      60

aggtaggaat tgctgaataa ttacctttag accagcctga gcaggagact tcccacgagg     120

gtgagctcat ttaatggttc ctaaaactca atcttgtgga tgtcagtggt catgcttgat     180

tatttgcagc agtgttttat g                                               201


<210>  20
<211>  201
<212>  DNA
<213>  homo sapiens

<400>  20
cataaaacac tgctgcaaat aatcaagcat gaccactgac atccacaaga ttgagtttta      60

ggaaccatta aatgagctca ccctcgtggg aagtctcctg ctcaggctgg tctaaaggta     120

attattcagc aattcctacc tgtgctttta ccttgttcct atacagtgtt tgaatttcat     180

acactggtca ggtcaacttt t                                               201


<210>  21
<211>  201
<212>  DNA
<213>  homo sapiens

<400> 21
agtcagatct cttagtatga tgatgacttg cctgtttaag attgtagtta tttgaactca 60

tgtctctaat gcctctttcg tgttttattg gactgtcatc ttcctagtat cttgtttcaa 120

ggcacagtga agctgtgtag cattcattga gcacaggaag agtttggtta tcttggatgc 180

agtaaagtgg atcagttggg c 201


<210> 22
<211> 201
<212> DNA
<213> homo sapiens

<400> 22
gcccaactga tccactttac tgcatccaag ataaccaaac tcttcctgtg ctcaatgaat 60

gctacacagc ttcactgtgc cttgaaacaa gatactagga agatgacagt ccaataaaac 120

acgaaagagg cattagagac atgagttcaa ataactacaa tcttaaacag gcaagtcatc 180

atcatactaa gagatctgac t 201


<210> 23
<211> 201
<212> DNA
<213> homo sapiens

<400> 23
ctgctcttca gtgccgtgcc agatttgcgg cagcgggagg ctaaggtggg tctttaaagt 60

atgttacact ttagttcctc attggccatg actcagctca ttagaacttt ctgttatttg 120

ggtggtgccc tcagcctaaa agagcctcct ctcccctccc ctggaaggtc ttggtcctcc 180

tccctgcggc ttttccctac t 201


<210> 24
<211> 201
<212> DNA
<213> homo sapiens

<400> 24
agtagggaaa agccgcaggg aggaggacca agaccttcca ggggagggga gaggaggctc 60

ttttaggctg agggcaccac ccaaataaca gaaagttcta atgagctgag tcatggccaa 120

tgaggaacta aagtgtaaca tactttaaag acccacctta gcctcccgct gccgcaaatc 180

tggcacggca ctgaagagca g 201


<210> 25
<211> 201
<212> DNA
<213> homo sapiens

<400> 25
gctgtgcaga aatggtgcta acatgctttc cgcgtgaagg acggaggcct agatgcctgg 60

ggcggtcacc gtgggtggaa gatgctcttg acaagcatct atattggtgc ctgggttcca 120

gggagctgca ccatgccgcg cttccattgt tccttatagc ctgatggggc tgcaagagct 180

tgcctgcatc tggattcctc t 201

<210> 26
<211> 201
<212> DNA
<213> homo sapiens

<400> 26
agaggaatcc agatgcaggc aagctcttgc agccccatca ggctataagg aacaatggaa 60

gcgcggcatg gtgcagctcc ctggaaccca ggcaccaata tagatgcttg tcaagagcat 120

cttccaccca cggtgaccgc cccaggcatc taggcctccg tccttcacgc ggaaagcatg 180

ttagcaccat ttctgcacag c 201

<210> 27
<211> 201
<212> DNA
<213> homo sapiens

<400> 27
cagacctgag gcccttgggg cctgtcactg tcctgctcga atctcttgct ccaatcctca 60

atatcgccca ctcactttct aactggagct tctgagagac caaaccctca ctggaggcag 120

cctcaggcct ctcagacctt caggggagcc tagaaggaag tgtttccagg acaggaggat 180

cctcacgggg ccccagttca c 201

<210> 28
<211> 201
<212> DNA
<213> homo sapiens

<400> 28
gtgaactggg gccccgtgag gatcctcctg tcctggaaac acttccttct aggctcccct 60

gaaggtctga gaggcctgag gctgcctcca gtgagggttt ggtctctcag aagctccagt 120

tagaaagtga gtgggcgata ttgaggattg gagcaagaga ttcgagcagg acagtgacag 180

gccccaaggg cctcaggtct g 201

<210> 29
<211> 201
<212> DNA
<213> homo sapiens

<400> 29
   atgacttttg gttaccaaca tttattgttt tttcttctga attatttcta ttttttttct 60
   attatttccc acattgggct tcagagggca ttagttctgc gggattttaa agagtagagg 120
   gcaaaaaaat tcaaagaacc aatatgttta agaaatgcca gtgttaacat tttttttaat 180
   ccttaagact agtcagacaa c 201

<210> 30
<211> 201
<212> DNA
<213> homo sapiens

<400> 30
gttgtctgac tagtcttaag gattaaaaa aatgttaaca ctggcatttc ttaaacatat 60

tggttctttg aatttttttg ccctctactc tttaaaatcc cgcagaacta atgccctctg 120

aagcccaatg tgggaaataa tagaaaaaaa atagaaataa ttcagaagaa aaaacaataa 180

atgttggtaa ccaaaagtca t 201

<210> 31
<211> 201
<212> DNA
<213> homo sapiens

<400> 31
gactgcactt gtttttgaaa aaaaaaatat gtatacacat atctccctgt ttaatacttt 60

ccttatctaa acacctcaaa ttgcttcagt ttgagtgatt ttgatgtttt aaatttcact 120

tgggtttaga gtctgctgga gtctcaacag atgccaaagc ataaaaaatg gtttgagtga 180

ttttgaatga ggaactgtaa t 201

<210> 32
<211> 201
<212> DNA
<213> homo sapiens

<400> 32
attacagttc ctcattcaaa atcactcaaa ccatttttta tgctttggca tctgttgaga 60

ctccagcaga ctctaaaccc aagtgaaatt taaaacatca aaatcactca aactgaagca 120

atttgaggtg tttagataag gaaagtatta aacagggaga tatgtgtata catatttttt 180

ttttcaaaaa caagtgcagt c 201

<210> 33
<211> 201
<212> DNA
<213> homo sapiens

<400> 33
tgtgcatgca cgtgtgtgtg tgtgtgtgtg tgtgtgtgtt caggagggag aggggttgtg 60

aatcaaggca ctgtaatgga aataataaat caattttgaa gagcttgagt aagatcattg 120

gaaaagtcta gctatatctg tttttggcta agtgtggctc cttcgttatt tcactaaatt 180

tcattatctc tgcaaggagt g 201

<210> 34
<211> 201

<212>  DNA
<213>  homo sapiens

<400>  34
cactccttgc agagataatg aaatttagtg aaataacgaa ggagccacac ttagccaaaa     60

acagatatag ctagactttt ccaatgatct tactcaagct cttcaaaatt gatttattat    120

ttccattaca gtgccttgat tcacaacccc tctccctcct gaacacacac acacacacac    180

acacacacac gtgcatgcac a                                              201


<210>  35
<211>  201
<212>  DNA
<213>  homo sapiens

<400>  35
tccttctttc cctacctgcc actttcatgt cctaaaacca ggtgttcaaa agcaagcact     60

atggccatgt tctccaaact ctgtgccaag atattctgtg gtgatgcagt gagctcaaag    120

aagtgccctg aaatatttaa tattttcagg ggaaacagca acactgcaca tacaagcttg    180

aggtaattta tagtttcaac a                                              201


<210>  36
<211>  201
<212>  DNA
<213>  homo sapiens

<400>  36
tgttgaaact ataaattacc tcaagcttgt atgtgcagtg ttgctgtttc ccctgaaaat     60

attaaatatt tcagggcact tctttgagct cactgcatca ccacagaata tcttggcaca    120

gagtttggag aacatggcca tagtgcttgc ttttgaacac ctggttttag gacatgaaag    180

tggcaggtag ggaaagaagg a                                              201


<210>  37
<211>  201
<212>  DNA
<213>  homo sapiens

<400>  37
tagagggagg aagtgatgta caggaaagtg ggtacatggt gtgctgggag gggggccttg     60

tagacaagat ggggacaatt gtaaacagaa gatcggggct aatgtaaaag tgcaagatcc    120

tcttctgggg acagccttct tgtcttcatg gatagtgcat atagaaaagc acccacgatt    180

cccagtcatt gaaaaccaga g                                              201


<210>  38
<211>  201
<212>  DNA
<213>  homo sapiens

<400>  38

ctctggtttt caatgactgg gaatcgtggg tgcttttcta tatgcactat ccatgaagac      60

aagaaggctg tccccagaag aggatcttgc acttttacat tagccccgat cttctgttta     120

caattgtccc catcttgtct acaaggcccc cctcccagca caccatgtac ccactttcct     180

gtacatcact tcctccctct a                                               201


<210>  39
<211>  201
<212>  DNA
<213>  homo sapiens

<400>  39
gccagaggta aaccaggtct tccagagtat accagaaagg actgcatgtt gtctgtttga      60

gtgggggact tcatgcccaa ttttcacagg gcaaagcatc acctggtgtc ttgtgtggca     120

aacagctttg atacttgccc atgtcccatg gtgtccagga gacttttcat taaatgaggt     180

gaccactgcc cctttagggc g                                               201


<210>  40
<211>  201
<212>  DNA
<213>  homo sapiens

<400>  40
cgccctaaag gggcagtggt cacctcattt aatgaaaagt ctcctggaca ccatgggaca      60

tgggcaagta tcaaagctgt ttgccacaca agacaccagg tgatgctttg ccctgtgaaa     120

attgggcatg aagtccccca ctcaaacaga caacatgcag tcctttctgg tatactctgg     180

aagacctggt ttacctctgg c                                               201

## Description

The present invention relates to an oligo- or polynucleotide which can be used in the diagnosis and therapy of restless legs syndrome (RLS). Furthermore, the invention relates to methods of diagnosing a predisposition for or RLS and a method for selecting a therapy to prevent and treat RLS. Moreover, a method for determining the dose of a drug used for treating a patient suffering from RLS and a method to test the efficacy of a drug used for treating RLS in treating other dopaminergic diseases, sleep disorders or spinocerebellar ataxias as well as the use of a drug effective in treating RLS for testing the efficacy of said drug in treating other dopaminergic diseases, sleep disorders or spinocerebellar ataxias. The invention also envisages methods of identifying an inhibitor, pro-drug or drug capable of modulating the activity of a peptide or protein of the invention, a diagnostic kit for detection of a single nucleotide polymorphism and gene therapies on the basis of gene variants detected with the present invention.

Several documents are cited throughout the text of this specification. The disclosure content of the documents cited herein (including manufacturer's specifications, instructions, etc.) is herewith incorporated by reference.

Nightwalkers, as RLS patients call themselves, are forced to move their legs during periods of rest especially in the evening and night to relieve uncomfortable or painful sensations in the deep of the calf. This diurnal variation leads to impaired sleep onset and the periodic leg movements during sleep in the vast majority of patients contribute to severe sleep disruption and a reduced quality of life as a major consequence of the disease (Allen et al., Sleep Med. 4, 101-119 (2003)). There are recognized secondary forms of RLS such as in iron deficiency, pregnancy, and end-stage renal disease and associated morbidity such as increased cardiovascular risk (Winkelmann et al., Sleep Med. 7, 545-552 (2006)). RLS is one of the most common neurological disorders with an age-dependent prevalence of up to 10% in the elderly in North America and Europe and affects twice as many females as males (Berger et al., Arch. Int. Med. 164, 196-202 (2004); Allen et al., Arch. lnt. Med. 165, 1286-1292 (2005)). Dopaminergic agents originally developed for Parkinson disease provide first line treatment leading to a prompt relief of RLS with an unknown mode of action (Hening et al., Sleep 27, 560-583 (2004)). Neurophysiological, pharmacological and neuroimaging studies suggest that the characteristic symptoms originate in the central nervous system, yet the underlying neurobiology remains obscure (Barriére et al., Prog. Neurobiol. 77, 139-165 (2005); Trenkwalder et al., Lancet Neurol. 4, 465-475 (2005)).
RLS is a highly heritable trait as evidenced by segregation analyses in several populations (Winkelmann et al., Ann. Neurol. 52, 297-302 (2002); Chen et al., Am. J. Hum. Genet. 74, 876-885 (2004); Mathias et al., Hum. Hered. 62, 157-164 (2006)). So far, genome-wide linkage studies in RLS families reported five loci based on recessive (RLS1) ( Desautels et al., Am. J. Hum. Genet. 69, 1266-1270 (2001)) or dominant inheritance models (RLS2-RLS5) (Chen et al., Am. J. Hum. Genet. 74, 876-885 (2004) ; Bonati et al., Brain 126, 1485-1492 (2003); Pichler et al., Am. J. Hum. Genet. 79, 716-723 (2006); Levchenko et al., Neurology 67, 900-901 (2006)) providing evidence that RLS is a genetically heterogeneous condition (Kemlink et al., Mov. Disord. 22, 207-212 (2007)). Despite the availability of extended families the success of linkage studies has been fairly limited (Rao et al., in Restless Legs Syndrome. Diagnosis and Treatment. (ed Ondo,W.G. ), 111-123 (Informa Healthcare, New York, 2007)).
Thus, the lack of identification of genes involved in the etiology of RLS have, up to date, precluded a more accurate diagnosis and treatment of RLS.

Therefore, the technical problem underlying the present invention was to provide alternative or improved means and methods for diagnosing or prognosing of RLS or a predisposition therefore.
The solution to this technical problem is achieved by providing the embodiments characterized in the claims.

Accordingly, the present invention relates to an oligo- or polynucleotide comprising or consisting of an oligo- or polynucleotide selected from the group consisting of:
(a) an oligo- or polynucleotide consisting of at least 10, particularly preferred at least 17 consecutive nucleotides of any of SEQ ID NOs: 1 to 40; wherein the at least 10 and particularly preferred at least 17 consecutive nucleotides contain a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40;
(b) an oligo- or polynucleotide hybridizing under stringent conditions to at least a portion of the oligo- or polynucleotide of (a), wherein said portion comprises the nucleotide in position 101 of any of SEQ ID NOs: 1 to 40, wherein said oligo- or polynucleotide contains a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; , a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40; and
(c) an oligo- or polynucleotide identical to the oligo- or polynucleotide of (a) or (b) with the exception that T is replaced by U.

In the context of the present invention the term "oligo- or polynucleotide" refers to nucleic acid molecules of different length: an oligonucleotide is a nucleic acid molecule consisting of up to 30 bp, a polynucleotide is a nucleic acid molecule consisting of more than 30 bp. The oligo- or polynucleotides comprise most preferably at least 17 nucleotides, but may also comprise at least 19, 25, 50, 100, 150, 200 or more nucleotides, whereas oligonucleotides of 10, 11, 12, 13, 14, 15 or 16 nucleotides are also envisaged. The present invention encompasses a large number of oligo- or polypetides, which all contain as a common denominator the nucleotide at position 101 of any of SEQ ID NO: 1 to 40. For example, the 17-mer of the invention contains as a common denominator the nucleotide at position 101 of any of SEQ ID NO: 1 to 40, wherein the said nucleotide at position 101 may be located within the 17-mer at position 1, 2, 3, 4, 5, 6, 7, 8, 9, 10, 11, 12, 13, 14, 15, 16 or 17 of the 17-mer if viewed from its 5'end. The oligo- or polynucleotides of the present invention are characterized as being associated with restless legs syndrome (referred to as RLS thereafter). The sequences of any of the SEQ ID NO: 1 to 40 were uniformly chosen to contain the 100bp upstream and the 100bp downstream of the respective SNP position at the genetic locus, hence the SNP is uniformly located at position 101 of any of SEQ ID NO: 1 to 40. Thus, the present invention not only encompasses the SNPs per se being an exchange of a nucleotide in comparison to the wild type sequence, but also the locus surrounding the SNPs being an oligo- or polynucleotide of the invention comprising or consisting of an oligo- or polynucleotide consisting of at least 10, particularly preferred at least 17 consecutive nucleotides of any of the specified SEQ ID NO: 1 to 40. Said SNPs can be unambiguously identified with information of position and the sequences contained in the SEQ ID NO: 1 to 40 as provided in the present application. The sequence of or other information on the genes MEIS1, BTBD9, MAP2K5, LBXCOR1, PTPRD or A2BP1 can readily be retrieved from the database maintained at the NCBI (http://www.ncbi.nlm.nih.gov/) using the following GenelDs: 4211 (MEIS1), 114781(BTBD9), 5607 (MAP2K5), 390598 (LBXCOR1), 5789 (PTPRD) or 54715 (A2BP1).

The term "RLS" as used in accordance with the present invention comprises all forms of symptoms currently scientifically associated with term "restless legs syndrome" such as, for example, RLS with intermittent discomfort, RLS with sleep disorder, acute and chronic RLS as well as secondary forms of RLS. The oligo- or polynucleotides of the invention include oligo- or polynucleotides that have at least 70%, preferably at least 75%, at least 80%, at least 85%, at least 90% or at least 95% sequence identity to an MEIS1, BTBD9, MAP2K5, LBXCOR1, PTPRD or A2BP1 gene. All oligonucleotides and polynucleotides of the invention, irrespective of their degree of identity, carry one of the nucleotides specified herein above at position 101 of the further specified SEQ ID NO: 1 to 40.

The term "identical" in the context of nucleic acid sequences refers to the residues in the two sequences which are the same when aligned for maximum correspondence, wherein correspondence means complementarity being the pairing of bases according to the Watson-Crick base pairing model; also included are other base pair models such as, for example Hoogsteen base pairing. The length of sequence identity comparison may be over a stretch of at least nine nucleotides, preferably at least 20 nucleotides, at least 24 nucleotides, at least 28 nucleotides, at least 32 nucleotides, and most preferably at least 36 nucleotides or more nucleotides. There are a number of different algorithms known in the art which can be used to measure nucleotide sequence identity. For instance, polynucleotide sequences can be compared using Fasta, a program in GCG Version 6.6. Fasta provides alignments and percent sequence identity of the regions of the best overlap between the query and the search sequence (Pearson, 1980, herein incorporated by reference). For instance, percent sequence identity between nucleic acid sequences can be determined using Fasta with its default parameters (a word size of 6 and the NOPAMfactor for the scoring matrix) as provided in GCG Version 6.1, herein incorporated by reference.

The term "hybridizing" as used herein refers to a pairing of an oligo- or polynucleotide to a (partially) complementary strand of this oligo- or polynucleotide which thereby form a hybrid. Said complementary strand oligo- or polynucleotides are, e.g. the oligo-or polynucleotides described in item (b), supra, or parts of oligo- or polynucleotides comprising at least 10, preferably at least 15 such as at least 25 consecutive nucleotides thereof, if used as primers or probes. Said complementary oligo- or polynucleotides may be useful as probes in Northern or Southern blot analysis of RNA or DNA preparations, PCRs and the like or primer extension protocols respectively. In this connection, item (b) refers to oligo- or polynucleotides the sequence of which is uniquely fitting to (hybridizing to/complementary to preferably 100%) the sequences of the oligo- or polynucleotides described in (a), but not to wild type sequences. Hybridizing oligo- or polynucleotides of the present invention to be used as a probe in Southern or Northern blot preferably comprise at least 100, more preferably at least 200, and most preferably at least 500 nucleotides in length.
As mentioned above, the oligo- or polynucleotides of the invention may, for example, be useful as probes in Northern or Southern Blot analysis of RNA or DNA preparations, respectively, or can be used as oligonucleotide primers in PCR analysis dependent on their respective size. Also comprised by the invention are hybridizing oligo- or polynucleotides which are useful for analysing DNA-Protein interactions via, e.g., electrophoretic mobility shift analysis (EMSA). Preferably, said hybridizing oligo-or polynucleotides comprise at least 10, more preferably at least 15 nucleotides in length while a hybridizing oligo- or polynucleotide of the present invention to be used as a probe preferably comprises at least 100, more preferably at least 200, or most preferably at least 500 nucleotides in length, wherein the single nucleotide polymorphisms (SNPs) of the present invention preferably have a central location. "Central" meaning most preferably that an equal number of nucleotides is located in the 5'- and 3' -direction adjacent to the position of the SNP. In a different preferred embodiment 60, 70, 80 or 90 percent of nucleotides are located in the 5'- or 3'-direction adjacent to the position of the SNP and the remaining nucleotides are located in the opposite direction.
It is well known in the art how to perform hybridization experiments with nucleic acid molecules, i.e. the person skilled in the art knows what hybridization conditions s/he has to use in accordance with the present invention. Such hybridization conditions are referred to in standard text books such as Molecular Cloning A Laboratory Manual, Cold Spring Harbor Laboratory (1989) N.Y. Preferred in accordance with the present invention are oligo- or polynucleotides which are capable of hybridizing to the oligo- or polynucleotides of the invention or parts thereof which are associated with RLS under stringent hybridization conditions, i.e. which do not cross hybridize to unrelated oligo-or polynucleotides such as oligo- or polynucleotides encoding a peptide or protein different from the peptides or proteins of the invention.
Nucleic acid hybridization will be affected by such conditions as salt concentration, temperature, or organic solvents, in addition to the base composition, length of the complementary strands and the number of nucleotide base mismatches between the hybridizing nucleic acids, as will be readily appreciated by those skilled in the art. Stringent temperature conditions will generally include temperatures in excess of 30°C, typically 37°C, and preferably in excess of 45°C. Stringent salt conditions will ordinarily be less than 1000 mM, typically less than 500 mM and preferably less than 200 mM. However, the combination of parameters is much more important than the measure of any single parameter; see, e.g., Wetmur and Davidson, 1968. Probe sequences may also hybridize specifically to duplex DNA under certain conditions to form triplex or higher order DNA complexes. The preparation of such probes and suitable hybridization conditions are well known in the art. Oligo- or polynucleotides which are capable of hybridizing to the oligo- or polynucleotides of the invention are preferably at least 70%, preferably at least 75%, at least 80%, at least 85%, at least 90% or at least 95% or 100% identical to the nucleic acid sequences of the invention.
Generally, stringent hybridization conditions refer to conditions which comprise, e.g. an overnight incubation at 42°C in a solution comprising 50% formamide, 5x SSC (750 mM NaCl, 75 mM sodium citrate), 50 mM sodium phosphate (pH 7.6), 5x Denhardt's solution, 10% dextran sulphate, and 20 µg/ml denatured, sheared salmon sperm DNA, followed by washing the filters in 0.1 x SSC at about 65°C. Said conditions for hybridization are also known by a person skilled in the art as "highly stringent conditions for hybridization". Also contemplated are nucleic acid molecules that hybridize to the oligo- or polynucleotides of the invention at lower stringency hybridization conditions ("low stringency conditions for hybridization"). Changes in the stringency of hybridization and signal detection are primarily accomplished through the manipulation of formamide concentration (lower percentages of formamide result in lowered stringency), salt conditions, or temperature. For example, lower stringency conditions include an overnight incubation at 37°C in a solution comprising 6X SSPE (20X SSPE = 3M NaCl; 0.2M NaH₂PO4; 0.02M EDTA, pH 7.4), 0.5% SDS, 30% formamide, 100 µg/ml salmon sperm blocking DNA; followed by washes at 50°C with 1X SSPE, 0.1% SDS. In addition, to achieve an even lower stringency, washes performed following stringent hybridization can be done at higher salt concentrations (e.g. 5X SSC). It is of note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Such modifications can generally be effected by the skilled person without further ado. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The embodiment recited herein above preferably refers to highly stringent conditions and alternatively to conditions of lower stringency.

The oligo- or polynucleotides of the present invention or the single nucleotide polymorphisms contained therein being associated with RLS can be used in a variety of ways as outlined in the methods and uses provided and described hereinafter. Those methods include inter alia diagnostic methods, methods to test drug efficacy with regard to the presence of the oligo- or polynucleotides of the invention or SNPs contained therein. A single nucleotide polymorphism (SNP) is a polymorphism of a single nucleotide or base. The SNP database maintained at NCBI (http://www.ncbi.nlm.nih.gov/SNP/) divides SNPs into SNPs in the proximity of a known locus and such that are 5' further away than 2 kb from the most 5' feature of a gene and 3' further away than 500 bases from the most 3' feature of a gene. SNPs in the proximity of a known locus are further divided into SNPs occurring at an mRNA location and such that do not. SNPs occurring at an mRNA location comprise coding and non-coding SNPs.

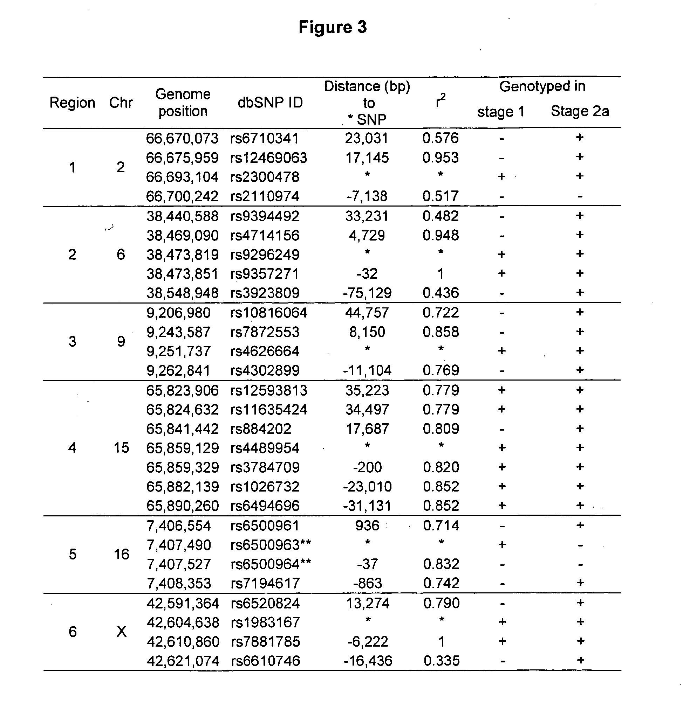

The described SNPs are most significantly associated with RLS. Figure 3 summarises the position of these SNPs on the chromosomes and in the genome, the region of the locus containing the SNP and the dbSNP ID.

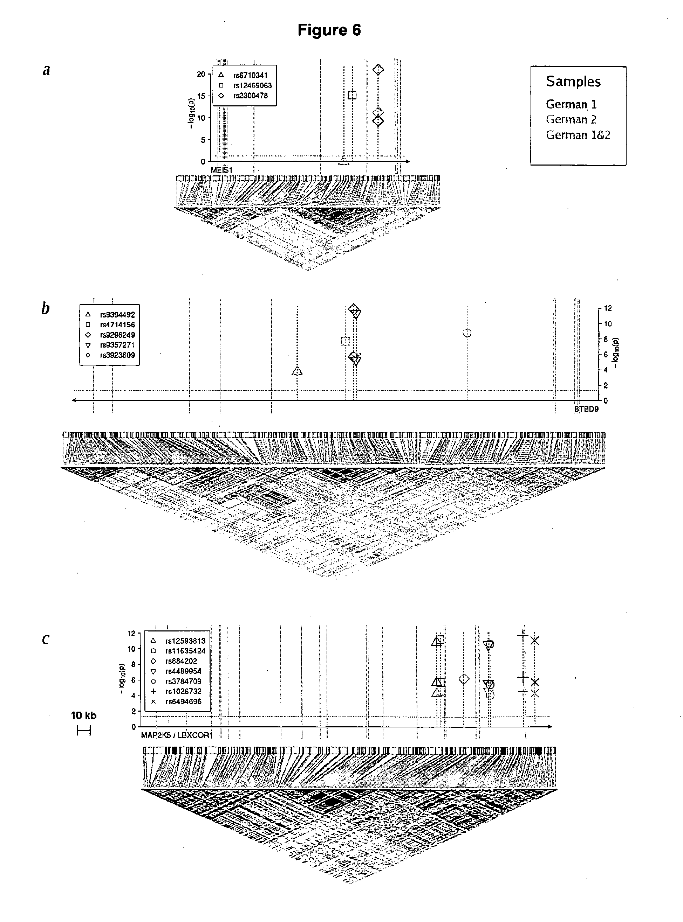

The increasing medical attention to RLS in recent years is matched by our ignorance about its underlying molecular basis. The present invention however, for the first time provides sequence variants associated with RLS. Previous efforts of investigations of large RLS families led to an increasing number of linkage signals with achieved LOD scores only between 2.5 and 3.2, well below the simulated maximum LOD (logarithm of the odds) score respective families. Numerous further family based linkage and candidate gene studies failed and only few of the identified loci have finally been replicated in independent families. This provides indirect evidence for the complexity of RLS genetics including a high phenocopy rate, intrafamilial allelic and non-allelic heterogeneity. Surprisingly and based on a large scale, two stage genomewide association study, the power to detect several strong effects in the range of an LOD up to 1.5 is demonstrated.
The identification of significant regions in 3 genes which have not been candidates based on prior art knowledge provides a complete new model about the natural history of RLS pathophysiology, namely its causative association with SNPs. Their further characterization will lead to a new understanding of the molecular basis of RLS.
The first of these regions was on 2p (short arm of chromosome 2) located in a 66 kb linkage disequilibrium block containing exon 8 and 9 of the MEIS1 gene (cf. Figure 6). Here two of three SNPs showed nominally significant evidence for association with P-values less than 10⁻¹¹. MEIS1 is a member of a family of highly conserved homeobox (Hox) genes which form heterodimers with PBX and have been found mutated in hematological cancers (Mamo et al., Blood 108, 622-629 (2006)). Now that the present invention has been completed several potential links to RLS emerge: During embryonic development MEIS1 is essential for distal limb formation (Mercader et al., Nature 402, 425-429 (1999)) and children with restless legs syndrome are often described as having growing pains (Rajaram et al., Sleep 27, 767-773 (2004)). In addition, MEIS1 is part of a Hox transcriptional regulatory network that specifies spinal motor neuron pool identity and connectivity, constituents of the central pattern generators (Dasen et al., Cell 123, 477-491 (2005)). Intriguingly spinal hyperexcitability is an established component in the genesis of periodic limb movements found in RLS subjects (Bara-Jimenez et al., Neurology 54, 1609-1615 (2000)). Specific functions of MEIS1 in postembryonic tissues have still to be established. The protein is known to be expressed in the adult mouse brain in cerebella granule cells, the forebrain, and, interestingly, in dopaminergic neurons of the substantia nigra (Allen Brain Atlas. *http:llwww. brain-map. org* (2004)).
The second significant region was on 6p (short arm of chromosome 6) located within a 54 kb linkage disequilibrium block in intron 5 of the BTBD9 gene (cf. Figure 6). All five SNPs tested showed nominally significant evidence for association. Four out of these five showed P-values less than 10⁻⁵. Little is known about BTBD9 other than that it belongs to the BTB(POZ) proteins. BTB stands for broad complex, tramtrack and bric à brac genes that in the Drosophila are required for embryonic development, cell fate determination in the eye, metamorphosis, and, interestingly, pattern formation in the limbs (Godt et al., Development 119, 799-812 (1993)). Functions of BTB(POZ) proteins include transcription repression, cytoskeleton regulation, tetramerization and gating of ion channels as well as protein ubiquitination/degradation (Stogios et al., Genome Biol. 6, R82 (2005)). The modular nature of this protein and the universal nature of the particular domains of BTBD9 make assignment of a specific function difficult at present.
The third region defined by seven SNPs tested on 15q (long arm of chromosome 15) showed significant evidence for association with P-values less than 10⁻⁴. This region contains a 95 kb linkage disequilibrium block of the 3'end of the MAP2K5 gene, a member of the mitogen-activated protein kinase family and the LBXCOR1 gene (cf. Figure 6). MAPK pathways are conserved from yeast to human and are activated by a signaling cascade that mediates the transduction of extracellular signals to cytoplasmatic nuclear effectors (Chen et al., Chem. Rev. 101, 2449-2476 (2001)). MAP2K5 is a specific upstream activator of ERK5 and this pathway is activated by oxidative stress, hyperosmolarity and growth factors. In addition, MAP2K5 and ERK5 are abundantly expressed in heart and skeletal muscles and the MAP2K5/ERK5 MAP kinase cascade is critical for early steps of muscle cell differentiation (Dinev et al., EMBO Rep. 2, 829-834 (2001)). The possible link between RLS risk alleles and known biological functions of the MAP2K5/ERK5 pathway is of particular interest since this pathway plays an important role in neuroprotection of dopaminergic neurons (Cavanaugh et al., J. Neurosci. Res. 84, 1367-1375 (2006)). It is also known that ERK5 elevates NURR1 transcriptional activity which is a potential substrate of this kinase (Sacchetti et al., Nucleic Acids Res. 34, 5515-5527 (2006)). NURR1 in turn is known to be an essential transcription factor for the acquisition and maintenance of the phenotype of dopamine-synthesizing neurons in the mesencephalon (Sachetti et al., J. Neurochem. 76, 1565-1572 (2001)) and has been implicated in Parkinson disease (Le et al., Nat. Genet. 33, 85-89 (2003)). LBXCOR1 annotated in the same LD block acts as transcriptional corepressor of the homeobox gene LBX1 which plays a critical role in the development of sensory pathways in the spinal cord that relay pain and touch (Gross et al., Neuron 34, 535-549 (2002)).
In addition, SNPs in two more genes showed nominal significance, four SNPs on chromosome 9 in the PTPRD gene and two SNPs on chromosome 16 in the A2BP1 gene. PTPRD is a receptor-type protein-tyrosine phosphatase expressed in the specialized regions of the brain, including the hippocampal CA2 and CA3, in B lymphocytes, and in the thymic medulla (Uetani et al., EMBO J. 19: 2775-2785, 2000). According to this document, PTPRD is an important regulator of synaptic plasticity and possibly in learning and memory. The possible connection to RLS is still unknown.

Shibata et al. (Hum. Molec. Genet. 9: 1303-1313, 2000) identified a novel protein, A2BP1 (ataxin-2-binding protein 1), which binds to the C terminus of ataxin-2. The 377-amino acid coding sequence contains an RNP motif that is highly conserved among RNA-binding proteins. Immunocytochemistry showed that A2BP1 was expressed in the cytoplasm of Purkinje cells and dentate neurons in a punctate pattern similar to that seen for ataxin-2 labeling. Again, the possible connection to RLS is still unknown.

Due to the high rate of heritability and general impact on the patient's life there has been a long-felt need for alleviating symptoms and minimizing the risk to develop RLS. This has over the last years provided motivation for intensive research in the field of restless legs syndrome, which has, however, prior to the present invention not led to results significantly impacting the diagnosis and outcome of the disease. The oligo- or polynucleotides and methods as disclosed in the present invention provide an important step in the improvement of clinical diagnosis and subsequent treatment of patients with RLS. Moreover, it also provides the possibility of prospective diagnosis of persons at risk such as, for example, persons having affected family members. Additionally, the invention provides the means and methods for successful treatment, prevention, and/or delay of RLS as will be discussed in further embodiments in detail infra.

In a preferred embodiment the present invention relates to an oligo- or polynucleotide of the present invention which is DNA.

The oligo- or polynucleotide of the invention according to this preferred embodiment may be, e.g., DNA, cDNA, genomic DNA or synthetically produced DNA or a recombinantly produced chimeric nucleic acid molecule comprising any of those oligo-or polynucleotides either alone or in combination. Preferably said oligo- or polynucleotide is part of a vector, particularly of plasmids, cosmids, viruses and bacteriophages used conventionally in genetic engineering that comprise an oligo- or polynucleotide of the invention. Such vectors may comprise further genes such as marker genes which allow for the selection of said vector in a suitable host cell and under suitable conditions.

In a further preferred embodiment, the oligo- or polynucleotide of the invention is a gene.

It is well known in the art that genes comprise structural elements which encode an amino acid sequence as well as regulatory elements which are involved in the regulation of the expression of said genes. Structural elements are represented by exons which may either encode an amino acid sequence or which may encode for RNA which is not encoding an amino acid sequence but is nevertheless involved in RNA function, e.g. by regulating the stability of the RNA or the nuclear export of the RNA.
Regulatory elements of a gene may comprise promoter elements or enhancer elements both of which could be involved in transcriptional control of gene expression. It is very well known in the art that a promoter is to be found upstream of the structural elements of a gene. Regulatory elements such as enhancer elements, however, can be found distributed over the entire locus of a gene. Said elements could reside, e.g., in introns, regions of genomic DNA which separate the exons of a gene. Promoter or enhancer elements correspond to polynucleotide fragments which are capable of attracting or binding polypeptides involved in the regulation of the gene comprising said promoter or enhancer elements. For example, polypeptides involved in regulation of said gene comprise the so called transcription factors.
Said introns may comprise further regulatory elements which are required for proper gene expression. Introns are usually transcribed together with the exons of a gene resulting in a nascent RNA transcript which contains both, exon and intron sequences. The intron encoded RNA sequences are usually removed by a process known as RNA splicing. However, said process also requires regulatory sequences present on a RNA transcript said regulatory sequences may be encoded by the introns.
In addition, besides their function in transcriptional control and control of proper RNA processing and/or stability, regulatory elements of a gene could be also involved in the control of genetic stability of a gene locus. Said elements control, e.g., recombination events or serve to maintain a certain structure of the DNA or the arrangement of DNA in a chromosome.
Single nucleotide polymorphisms can occur in exons of a gene which encode an amino acid sequence as discussed supra as well as in regulatory regions which are involved in the above discussed process. The analysis of the nucleotide sequence of a gene locus in its entirety including, e.g., introns may in light of the above be desirable. The polymorphisms comprised by the oligo- or polynucleotides of the present invention are involved in the etiology of RLS via mechanisms involving enhanced or reduced transcription of the MEIS1, BTBD9, MAP2K5, LBXCOR1, PTPRD or A2BP1 gene, stabilization of the gene's RNA transcripts, alteration of the processing of the primary RNA transcripts or posttranslational processing.

In a particularly preferred embodiment, the invention relates to the oligo- or polynucleotide of the present invention wherein the gene is MEIS1, BTBD9, MAP2K5, LBXCOR1, PTPRD or A2BP1.

In a further embodiment, the present invention relates to a vector comprising the oligo-or polynucleotide of the present invention.

Preferably, the vector is a plasmid, cosmid, virus, bacteriophage or another vector used e.g. conventionally in genetic engineering. The oligo- or polynucleotide of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, plZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).
The oligo- or polynucleotide of the present invention referred to above may also be inserted into vectors such that a translational fusion with another oligo- or polynucleotide is generated. The other oligo- or polynucleotide may encode a protein which may e.g. increase the solubility and/or facilitate the purifcation of the fusion protein. Non-limiting examples include pET32, pET41, pET43. The vectors may also contain an additional expressible oligo- or polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®. For vector modification techniques, see Sambrook and Russel (2001), loc. cit. Generally, vectors can contain one or more origin of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

The vector may further comprise nucleotide sequences encoding secretion signals as further regulatory elements. Such sequences are well known to the person skilled in the art. Depending on the expression system used, leader sequences capable of directing the expressed polypeptide to a cellular compartment may be added to the coding sequence of the polynucleotide of the invention. Such leader sequences are well known in the art.

Possible examples for regulatory elements ensuring the initiation of transcription comprise the cytomegalovirus (CMV) promoter, SV40-promoter, RSV-promoter (Rous sarcome virus), the lacZ promoter, the gai10 promoter, human elongation factor 1α-promoter, CMV enhancer, CaM-kinase promoter, the Autographa californica multiple nuclear polyhedrosis virus (AcMNPV) polyhedral promoter or the SV40-enhancer. For the expression in prokaryotes, a multitude of promoters including, for example, the tac-lac-promoter, the lacUV5 or the trp promoter, has been described. Examples for further regulatory elements in prokaryotes and eukaryotic cells comprise transcription termination signals, such as SV40-poly-A site or the tk-poly-A site or the SV40, lacZ and AcMNPV polyhedral polyadenylation signals, downstream of the polynucleotide.

Furthermore, the vector of the invention preferably comprises a selectable marker. Examples of selectable markers include neomycin, ampicillin, and hygromycine, kanamycine resistance and the like. Specifically-designed vectors allow the shuttling of DNA between different hosts, such as bacteria- fungal cells or bacteria-animal cells (e. g. the Gateway® system available at Invitrogen).

An expression vector according to this invention is capable of directing the replication, and the expression, of the oligo- or polynucleotide and encoded protein of this invention. Suitable expression vectors which comprise the described regulatory elements are known in the art such as Okayama-Berg cDNA expression vector pcDV1 (Pharmacia), pRc/CMV, pcDNA1, pcDNA3 (In-Vitrogene, as used, inter alia in the appended examples), pSPORT1 (GIBCO BRL) or pGEMHE (Promega), or prokaryotic expression vectors, such as lambda gt11, pJOE, the pBBR1-MCS -series, pJB861, pBSMuL, pBC2, pUCPKS, pTACT1 or, preferably, the pET vector (Novagen).

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). For recombinant expression and secretion, the polynucleotide of interest may be ligated between e.g. the PelB leader signal, which directs the recombinant protein in the periplasm and the gene III in a phagemid called pHEN4 (described in Ghahroudi et al, 1997, FEBS Letters 414:521-526). Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). Mammalian host cells that could be used include, human Hela, 293, H9 and Jurkat cells, mouse NIH3T3 and C127 cells, Cos 1, Cos 7 and CV1, quail QC1-3 cells, mouse L cells and Chinese hamster ovary (CHO) cells. Alternatively, the recombinant (poly)peptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al.1991, Biochem J. 227:277-279; Bebbington et al. 1992, Bio/Technology 10:169-175). Using these markers, the mammalian cells are grown in selective medium and the cells with the highest resistance are selected. As indicated above, the expression vectors will preferably include at least one selectable marker. Such markers include dihydrofolate reductase, G418 or neomycin resistance for eukaryotic cell culture and tetracycline, kanamycin or ampicillin resistance genes for culturing in *E. coli* and other bacteria. Representative examples of appropriate hosts include, but are not limited to, bacterial cells, such as *E. coli,* Streptomyces and *Salmonella typhimurium* cells; fungal cells, such as yeast cells; insect cells such as Drosophila S2 and Spodoptera Sf9 cells; animal cells such as CHO, COS, 293 and Bowes melanoma cells; and plant cells. Appropriate culture mediums and conditions for the above-described host cells are known in the art.
Preferably said vector is an expression vector and/or a gene transfer or targeting vector. Expression vectors and gene targeting or transfer vectors are well-known in the art and can be adapted for specific purposes of the invention by the person skilled in the art. Thus, expression vectors derived from viruses such as retroviruses, vaccinia viruses, adeno-associated virus, herpes virus, or bovina papilloma virus, may be used for delivery of the polynucleotides or vectors of the invention into targeted cell populations. Methods which are well known to those skilled in the art can be used to construct recombinant viral vectors; see, for example, the techniques described in Sambrook, Molecular Cloning, A Laboratory Manual, Cold Spring Harbor Laboratory (1989) N.Y. and Ausubel, Current Protocols in Molecular Biology, Green Publishing Associates and Wiley Interscience, N.Y. (1889). Alternatively, the polynucleotides and vectors of the invention can be reconstituted into liposomes for delivery to target cells.

Transcriptional regulatory elements (parts of an expression cassette) ensuring expression in prokaryotes or eukaryotic cells are well known to those skilled in the art.

These elements comprise regulatory sequences ensuring the initiation of the transcription (e. g., translation initiation codon, promoters, enhancers, and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers, and/or naturally-associated or heterologous promoter regions. The expression control sequences linked to the oligo- or polynucleotide can e.g. be synthesized by standard methods, or isolated from natural sources. Ligation of the oligo- or polynucleotide to said transcriptional regulatory elements can be carried out using established methods. Moreover, the nucleic acid molecules of the invention may be designed for direct introduction or for introduction via liposomes, phage vectors or viral vectors (e.g. adenoviral, retroviral) into the cell. Additionally, baculoviral systems or systems based on Vaccinia Virus or Semliki Forest Virus can be used as eukaryotic expression system for the oligo- or polynucleotide of the invention.

In another embodiment, the present invention relates to a host cell genetically engineered with the oligo- or polynucleotide or the vector of the invention.

Said host cell may be a prokaryotic or eukaryotic cell; see supra. The oligo- or polynucleotide or vector of the invention which is present in the host cell may either be integrated into the genome of the host cell or it may be maintained extra-chromosomally. In this respect, it is also to be understood that the oligo- or polynucleotide of the invention can be used for "gene targeting" and/or "gene replacement", for restoring a mutant gene or for creating a mutant gene via homologous recombination; see for example Mouellic, Proc. Natl, Acad. Sci. USA, 87 (1990), 4712-4716; Joyner, Gene Targeting, A Practical Approach, Oxford University Press. Preferably, the host cell is a mammalian cell, a fungal cell, a plant cell, an insect cell or a bacterial cell. Preferred fungal cells are, for example, those of the genus Saccharomyces, in particular those of the species S. cerevisiae. The term "prokaryotic" is meant to include all bacteria which can be transformed or transfected with a polynucleotide for the expression of the proteins of the present invention. Prokaryotic hosts may include gram negative as well as gram positive bacteria such as, for example, *E. coli, S. typhimurium, Serratia marcescens* and *Bacillus subtilis.* Methods for preparing fused, operably linked genes and expressing them in bacteria or animal cells are well-known in the art (Maniatis, et al., Molecular Cloning: A Laboratory Manual, Cold Spring Harbor, N.Y., 1998). The genetic constructs and methods described therein can be utilized for expression of the protein of the present invention in prokaryotic hosts. In general, expression vectors containing promoter sequences which facilitate the efficient transcription of the inserted polynucleotide are used in connection with the host. The expression vector typically contains an origin of replication, a promoter, and a terminator, as well as specific genes which are capable of phenotypic selection of the transformed cells. The transformed prokaryotic host can be grown in fermentors and cultured according to techniques known in the art to achieve optimal cell growth. The polypeptides of the present invention can the be isolated from the grown medium, cellular lysates, or cellular membrane fractions. The isolation or purification of the microbially or otherwise expressed proteins or peptides of the invention may be by any conventional means such as, for example, preparative chromatographic separations and immunological separations such as those involving the use of polyclonal or monoclonal antibodies.

Thus, in a further embodiment the invention relates to a method for the production of a protein or peptide encoded by the oligo- or polynucleotide of the invention comprising culturing the host of the invention under conditions allowing the expression of the protein and recovering the protein or peptide.

A large number of suitable methods exists in the art to produce proteins (or fusion proteins) in appropriate hosts. If the host is a unicellular organism such as a prokaryote, a mammalian or insect cell, the person skilled in the art can revert to a variety of culture conditions. Conveniently, the produced protein is harvested from the culture medium, lysates of the cultured organisms or from isolated (biological) membranes by established techniques. In the case of a multicellular organism, the host may be a cell which is part of or derived from a part of the organism, for example said host cell may be the harvestable part of a plant. A preferred method involves the recombinant production of protein in hosts as indicated above. For example, nucleic acid sequences comprising the oligo- or polynucleotide according to the invention can be synthesized by PCR, inserted into an expression vector. Subsequently a suitable host may be transformed with the expression vector. Thereafter, the host is cultured to produce the desired protein(s), which is/are isolated and purified.

An alternative method for producing the protein of the invention is *in vitro* translation of mRNA. Suitable cell-free expression systems for use in accordance with the present invention include rabbit reticulocyte lysate, wheat germ extract, canine pancreatic microsomal membranes, *E*. *coli* S30 extract, and coupled transcription/translation systems such as the TNT-system (Promega). These systems allow the expression of recombinant polypeptides upon the addition of cloning vectors, DNA fragments, or RNA sequences containing coding regions and appropriate promoter elements.
In addition to recombinant production, fragments of the protein, the fusion protein or fragments of the invention may e.g. be produced by direct peptide synthesis using solid-phase techniques (cf Stewart et al. (1969) Solid Phase Peptide Synthesis; Freeman Co, San Francisco; Merrifield, J. Am. Chem. Soc. 85 (1963), 2149-2154).

Protein isolation and purification can be achieved by any one of several known techniques; for example and without limitation, ion exchange chromatography, gel filtration chromatography and affinity chromatography, high pressure liquid chromatography (HPLC), reversed phase HPLC, and preparative disc gel electrophoresis. Protein isolation/purification techniques may require modification of the proteins of the present invention using conventional methods. For example, a histidine tag can be added to the protein to allow purification on a nickel column. Other modifications may cause higher or lower activity, permit higher levels of protein production, or simplify purification of the protein.

In yet another embodiment the present invention relates to a peptide or protein encoded by the oligo- or polynucleotide of the invention or produced by the method of the present invention.

In this context it is also understood that the variant peptide or protein of the invention can be further modified by conventional methods known in the art. By providing said variant proteins according to the present invention it is also possible to determine the portions relevant for their biological activity or inhibition of the same. The terms "peptide" and "protein" are defined as follows: A peptide relates to an amino acid chain of not more than 30 consecutive amino acids, a protein is made up of an amino acid chain of more than 30 consecutive amino acids. The term "protein" is interchangeably used in connection with this invention with the term "polypeptide". Both terms confer the same meaning. Moreover, what is comprised by said terms is standard textbook knowledge.

In a further embodiment the invention relates to an antibody which binds specifically to the protein of the present invention.

The term "antibody" refers to intact molecules as well as fragments thereof, such as Fab, F(ab')₂, scFv, which are capable of binding an epitopic or antigenic determinant. The term "binds specifically" refers to the interaction between polypeptide and a binding molecule, such as agonist, an antagonist, or an antibody. The interaction is dependent upon presence of the aforementioned epitopic or antigenic determinant of the protein that is recognized by the binding molecule.

Antibodies against the wild type or genetic variants carrying the SNPs of the present invention of MEIS1, BTBD9, MAP2K5, LBXCOR1, PTPRD or A2BP1 protein of the invention can be prepared by well known methods using a purified protein according to the invention or a (synthetic) fragment derived therefrom as an antigen. Monoclonal antibodies can be prepared, for example, by the techniques as originally described in Köhler and Milstein, Nature 256 (1975), 495 - 497, and Galfré, Meth. Enzymol. 73 (1981), 3, which comprises the fusion of mouse myeloma cell to spleen cells derived from immunized mammals. The antibodies can be monoclonal antibodies, such as Fab, Fv or scFv fragments etc. Furthermore, antibodies or fragments thereof to the aforementioned polypeptides can be obtained by using methods which are described, e.g., in Harlow and Lane "Antibodies, A Laboratory Manual", CSH Press, Cold Spring Harbor, 1988. These antibodies can be used, for example, for immunohistochemistry and immunolocalization of the genetic variant MEIS1, BTBD9, MAP2K5, LBXCOR1, PTPRD or A2BP1 proteins. Advantageously, the antibody recognizes an epitope containing one or more amino acid substitution(s), deletion(s), addition(s) of the present invention and/or a combination thereof. The term "specifically recognizing" as used in accordance with the present invention means that the antibody does not or essentially does not cross-react with an epitope of similar structure. Cross-reactivity of antibodies may be tested, for example, by assessing binding of said antibodies under conventional conditions to the epitope of interest as well as to a number of more or less (structurally and/or functionally) closely related epitopes. Only those antibodies that bind to the epitope of interest in its relevant context (e.g. a specific motif in the structure of a protein) but do not or not essentially bind to any other epitope are considered specific for the epitope of interest and thus to be antibodies in accordance with this invention. Corresponding methods are described e.g. in Harlow and Lane, 1988 and 1999, loc cit.

In another embodiment, the present invention relates to a method of diagnosing RLS or a predisposition for RLS comprising determining (a) the presence of an oligo- or polynucleotide of the invention or (b) the presence of an SNP within the oligo- or polynucleotide of the invention as compared to the wild type sequence in a sample from a subject, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40 and wherein the presence of said oligo- or polynucleotide or said SNP is indicative of RLS or a predisposition for RLS.

The term "predisposition for RLS " as used in accordance with the present invention relates to a susceptibility, preferably to a genetic susceptibility to develop RLS.

The term "sample" refers to a biological sample, such as, for example, cells, tissues, or fluids (including serum, whole blood, cerebrospinal fluid, lymph, saliva, milk, pus, urine) to be isolated from an individual or from cell culture constituents. Any tissue or liquid sample obtained from a patient and/or (human) subject can be used for the detection of an oligo- or polynucleotide of the invention or the presence of an SNP within the oligo- or polynucleotide of the invention. It is a well known fact that genomic DNA of individuals, which harbours the individual genetic makeup of all genes, including the MEIS1, BTBD9, MAP2K5, LBXCOR1, PTPRD or A2BP1 gene can easily be purified from individual blood samples. Thus, a preferred sample to detect the oligo- or polynucleotide of the invention or the presence of said SNP within the oligo-or polynucleotide of the invention is blood. Methods for preparing the sample for genomic DNA extraction are well known in the art, and can be carried out using commercially available kits such as, for example, FlexiGene DNA Kit (Qiagen), or Qiagen Mini Prep Kit (Qiagen).

In accordance with this embodiment of the present invention, the method of testing the status of a disorder or susceptibility to such a disorder can be effected by using an oligo- or polynucleotide of the invention, e.g., in the form of a Southern or Northern blot or *in situ* analysis. Said oligo- or polynucleotide may hybridize to an oligo- or polynucleotide of the invention. Additionally, said testing can be done in conjunction with an actual blocking, e.g., of the transcription of the gene and thus is expected to have therapeutic relevance. Furthermore, an oligo- or polynucleotide can also be used for hybridizing to an mRNA corresponding to the oligo- or polynucleotide of the invention. The nucleic acids used for hybridization can, of course, be conveniently labeled by incorporating or attaching, e.g., a radioactive or other marker. Such markers are well known in the art. The labeling of said nucleic acid molecules can be effected by conventional methods.
Additionally, the presence or expression of the oligo- or polynucleotide of the present invention can be monitored by using a primer pair that specifically hybridizes to either of the corresponding nucleic acid sequences and by carrying out a PCR reaction according to standard procedures. Specific hybridization of the above mentioned probes or primers preferably occurs at stringent hybridization conditions. The term "stringent hybridization conditions" is well known in the art; see, for example, Sambrook et al., "Molecular Cloning, A Laboratory Manual" second ed., CSH Press, Cold Spring Harbor, 1989; "Nucleic Acid Hybridization, A Practical Approach", Hames and Higgins eds., IRL Press, Oxford, 1985. Sequence analysis may be performed using several techniques well known to the person skilled in the art. For example, the dye termination method can be used. The methods and mechanisms underlying the dye termination method are well known in the art and described (F. Sanger et al. (1977), DNA sequencing with chain-terminating inhibitors. Proc Natl Acad Sci U S A 74:5463-7). Furthermore, the mRNA, cRNA, cDNA or genomic DNA obtained from the subject may be sequenced to identify mutations which may be characteristic fingerprints of mutations in the oligo- or polynucleotide of the invention. The present invention further comprises methods wherein such a fingerprint may be generated by RFLPs of DNA or RNA obtained from the subject, optionally the DNA or RNA may be amplified prior to analysis, the methods of which are well known in the art. RNA fingerprints may be performed by, for example, digesting an RNA sample obtained from the subject with a suitable RNA-enzyme, for example RNase T₁, RNase T₂ or the like or a ribozyme and, for example, electrophoretically separating and detecting the RNA fragments as described above.
Further modifications of the above-mentioned embodiment of the invention can be easily devised by the person skilled in the art, without any undue experimentation.
The identification of polymorphisms associated with RLS is important for the prediction of the onset, the clinical course and/or severity of symptoms and subsequently for therapy outcome, including side effects of medications. Therefore, analysis of the oligo- or polynucleotides of the invention or SNPs within the oligo- or polynucleotides of the invention indicative of RLS, is a valuable tool for therapy with drugs and due to the present invention, has now become possible.

SNPs can be detected using a variety of methods well-known in the art. For example, hybridization based methods (Itakura et al., Ann Rev Biochem (1984), 53; 323-356) or PCR based methods can be employed. PCR is well known in the art and is employed to make large numbers of copies of a target sequence. This is done on an automated cycler device, which can heat and cool containers with the reaction mixture in a very short time. The PCR, generally, consists of many repetitions of a cycle which consists of: (a) a denaturing step, which melts both strands of a DNA molecule and terminates all previous enzymatic reactions; (b) an annealing step, which is aimed at allowing the primers to anneal specifically to the melted strands of the DNA molecule; and (c) an extension step, which elongates the annealed primers by using the information provided by the template strand. Generally, PCR can be performed for example in a 50 µl reaction mixture containing 5 µl of 10 x PCR buffer with 1.5 mM MgCl_{2;} 200 µM of each deoxynucleoside triphosphate, 0.5 µl of each primer (10 µM), about 10 to 100ng of template DNA and 1 to 2.5 units of Taq Polymerase. The primers for the amplification may be labeled or be unlabeled. DNA amplification can be performed, e.g., with a model 2400 thermal cycler (Applied Biosystems, Foster City, CA): 2 min at 94°C, followed by 30 to 40 cycles consisting of annealing (e. g. 30 s at 50°C), extension (e. g. 1 min at 72°C, depending on the length of DNA template and the enzyme used), denaturing (e. g. 10 s at 94°C) and a final annealing step at 55°C for 1 min as well as a final extension step at 72°C for 5 min. Suitable polymerases for use with a DNA template include, for example, E. coli DNA polymerase I or its Klenow fragment, T4 DNA polymerase, Tth polymerase, Taq polymerase, a heat-stable DNA polymerase isolated from Thermus aquaticus Vent, Amplitaq, Pfu and KOD, some of which may exhibit proof-reading function and/or different temperature optima. However, the person skilled in the art knows how to optimize PCR conditions for the amplification of specific nucleic acid molecules with primers of different length and/or composition or to scale down or increase the volume of the reaction mix.
A PCR based method to detect SNPs, for example, uses primers, wherein one primer hybridizes specifically to the region on the target sequence carrying the SNP and wherein said primer is exactly complementary to the target sequence. According to that method applied to the present invention, amplification of the target sequence will only occur, if said target sequence carries an SNP as defined in the oligo- or polnucleotide sequences of the invention. This is because the oligo- or polynucleotide will under stringent hybridization conditions not hybridize to the wild type sequence (with the consequence that no amplification product is obtained) but only to the sequence carrying said SNP. In one embodiment, said primer carries the complementary portion of the oligo- or polynucleotide containing the SNP in a central position. "Central" meaning most preferably that an equal number of nucleotides are located in the 5'- and 3'- direction adjacent to the position of the SNP. In different embodiments 100, 90, 80, 70 and 60 percent of nucleotides are located in the 5'- or 3'- direction adjacent to the position of the SNP and remaining nucleotides are located in the opposite direction. Also encompassed are embodiments were the primer sequence ends in the position complementary to the SNP. OF course, primers complementary to the wild type sequence can also be used wherein an amplification product is indicative of a situation where noc RLS-associated nucleotide exchange is present.
Also envisaged is the use of a primer extension assay wherein the target sequence is annealed to a primer complementary to the region adjacent to the SNP site. Dideoxyribonucleotides (ddNTPs) and DNA polymerase are added to the mixture and the primer is extended by a single nucleotide. The single nucleotide added is dependent on the allele of the amplified DNA. Primer extension biochemistry can be coupled with a variety of detection schemes, comprising fluorescence, fluorescence polarization (FP), luminescence and mass spectrometry (MS) (Li et al., Electrophoresis (1999), 20; 6, 1258-1265). Primers can be designed by methods well-known in the art including, for example, publicly available programs as primer3 (http://frodo.wi.mit.edu/). Preferred primers to be used according to the methods of the invention are primers that specifically hybridize to the oligo- or polynucleotides of the invention. Also preferred are primers that amplify the oligo- or polynucleotides of the invention without specifically hybridizing to said oligo- or polynucleotides of the invention. Furthermore, Restriction Fragment Length Polymorphism (RFLP) can be used to detect SNP. RFLP is a technique in which species, strains, or, as envisaged by the inventors, polymorphic alleles may be differentiated by analysis of patterns derived from cleavage of their DNA. If two organisms differ in the distance between sites of cleavage of a particular restriction endonuclease, the length of the fragments produced will differ when the DNA is digested with a restriction enzyme and can be analyzed using various methods including, for example, gelelectrophoresis.

The above definitions apply mutatis mutandis to the methods described in the following.

A preferred embodiment of the above method further comprises determining the presence of a peptide or protein of the invention or an amino acid exchange within the peptide or protein that is a consequence of an SNP as defined above within the oligo-or polynucleotide of the invention as compared to the wild type sequence.

In addition to methods described supra, which can be used to indirectly determine the presence of said peptide or protein or an said amino acid exchange within the peptide or protein that is a consequence of an SNP within the oligo- or polynucleotide, determining the presence can further be effected by employing an antibody of the invention or a fragment thereof. The antibody used may be labeled with detectable tags such as a histidine tags or a biotin molecule. Further methods to detect proteins or peptides of the invention are well known in the art, such as, for example immunohistochemistry.

In a further embodiment the invention relates to a method of diagnosing RLS or a predisposition for RLS comprising determining (a) the presence of a peptide or protein of the invention or (b) the presence of an SNP within the oligo- or polynucleotide of the invention as compared to the wild type sequence in a sample from a subject, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40 and wherein the presence of said peptide or protein or said SNP is indicative of RLS or a predisposition for RLS.

In a preferred embodiment of the above methods, said determining of said peptide or protein or the presence of said SNP comprises PCR based techniques, RFLP-based techniques, DNA sequencing-based techniques, hybridization techniques, single-strand conformation polymorphism analysis (SSCA), denaturating gradient gel electrophoresis (DGGE), mismatch cleavage detection, heteroduplex analysis, techniques based on mass spectroscopy, HPLC-based techniques, primer extension-based techniques, and 5'-nuclease assay-based techniques.
Said techniques are well known in the art.

A preferred embodiment of the above methods further comprises detecting an alteration in the expression of the peptide or protein of the invention or wild type MEIS1, BTBD9, MAP2K5, LBXCOR1, PTPRD or A2BP1 protein, wherein the alteration in expression results in either a higher amount or lower amount of peptide or protein compared to the amount of peptide or protein expression in the control sample, wherein the alteration of expression is indicative of RLS or a predisposition for RLS.

The gene expression levels of the peptide or protein of the invention or wild type MEIS1, BTBD9, MAP2K5, LBXCOR1, PTPRD or A2BP1 protein of the invention can be measured by any method known that can provide quantitative information regarding the levels to be measured. The methods preferably are highly sensitive and provide reproducible results. In particular, methods based upon the polymerase chain reaction (real-time PCR) and related amplification technologies, such as NASBA and other isothermal amplification technologies, may be used. A suitable approach may be, for example, real-time PCR employing the relative quantification approach using GAPDH or β-actin as housekeeping gene.

Amounts of purified protein in solution can be determined by physical methods, e.g. photometry. Methods of quantifying a particular protein in a mixture rely on specific binding, e.g of antibodies. Specific detection and quantitation methods exploiting the specificity of antibodies comprise immunohistochemistry (in situ) and surface plasmon resonance. Western blotting combines separation of a mixture of proteins by electrophoresis and specific detection with antibodies.

Detection of an alteration in expression of the peptide or protein of the invention or wild type MEIS1, BTBD9, MAP2K5, LBXCOR1, PTPRD or A2BP1 protein as well as detecting the presence of SNP as defined above within the oligo- or polynucleotide of the invention provides valuable insight in the relative distribution of the disease associated gene products and subsequently allows for a therapy taking account of the detected imbalance of said peptide or protein. The detection of peptides or proteins or SNPs within peptides or proteins as such have been described before and are well known to the person skilled in the art. The same applies mutatis mutandis to the methods below.

In yet another embodiment, the invention relates to a method of diagnosing RLS or a predisposition for RLS comprising detecting an alteration in the expression of the peptide or protein of the invention or wild type MEIS1, BTBD9, MAP2K5, LBXCOR1, PTPRD or A2BP1 protein, wherein the alteration in expression results in either a higher amount or lower amount of peptide or protein compared to the amount of peptide or protein expression in the control sample, wherein the alteration of expression is indicative of RLS or a predisposition for RLS.

In a preferred embodiment of the above methods, the alteration in expression is detected on the basis of levels of mRNA encoding the peptide or protein of the invention or wild type MEIS1, BTBD9, MAP2K5, LBXCOR1, PTPRD or A2BP1 protein.

Methods for detection of an alteration in expression on the mRNA level have been described above and further methods well known in the art can be applied without undue degree of further experimentation.

In a different embodiment, the invention relates to a method for selecting a therapy to prevent and/or treat RLS, wherein said method comprises:
(a) determining the presence or absence of an oligo- or polynucleotide of the invention or the presence or absence of an SNP within the oligo- or polynucleotide of the invention as compared to the wild type sequence in a sample obtained from a subject, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40; and
(b) selecting a therapy for said subject based on the results obtained in (a).

As is evident to the person skilled in the art, the genetic knowledge deduced from the present invention can now be used to exactly and reliably characterize the genotype of a patient as far as it is relevant in the diagnosis of RLS. Advantageously, RLS can be predicted and preventive or therapeutical measures can be applied accordingly. Moreover in accordance with the foregoing, in cases where a given drug takes an unusual effect, for example augmentation (cf. infra), a suitable individual therapy can be designed based on the knowledge of the individual genetic makeup of a subject with respect to the oligo- or polynucleotides of the invention and improved therapeutics can be developed as will be further discussed below.
A variety of therapies exists to treat RLS. Generally, physicians choose from dopamine agonists, benzodiazepine areceptor agonists, opioids, L-Dopa and anticonvulsants. Dopaminergic agents, largely used to treat Parkinson's disease, have been shown to reduce RLS symptoms and PLMD and are considered the initial treatment of choice. Good short-term results of treatment with levodopa plus carbidopa and combinations of levodopa and benserazid have been reported, although most patients eventually will develop augmentation, meaning that symptoms are reduced at night but begin to develop earlier in the day than usual. Dopamine agonists such as pergolide mesylate, pramipexole, and ropinirole hydrochloride may be effective in some patients and are less likely to cause augmentation. According to the invention, patients can be divided into subsets according to their individual genetic makeup being the presence of oligo- or polynucleotides of the invention or the presence of an SNP within the oligo- or polynucleotide of the invention as compared to the wild type sequence of the mentioned genes. These subsets of patients may react differently to the identical drug applied in the identical dosage regimen with regard to the onset of, the extent of and the duration of beneficial effects and/or side-effects. Thus, due to the invention, if the pharmacological effects of a drug with regard to the specific genetic set-up in the mentioned genes are known, first a suitable drug and second a suitable dosage regimen can be selected having overall a more beneficial effect than standard therapeutic approaches. The pharmacologic effects of drugs can be determined by methods well known in the art and includes for example in vitro methods as described below or collecting data that refers to an alleviation of symptoms in said patient or group of patients.

Furthermore, a preferred embodiment of the above method is disclosed wherein after step (a) and prior to step (b) in step (a') the ability to develop augmentation under L-Dopa or dopamine agonist therapy is determined; and wherein step (b) comprises selecting a therapy for said subject based on the results obtained in (a) and (a').

Augmentation is considered to be the most important complication of L-Dopa or dopaminergic therapies for RLS. Augmentation describes an earlier onset of RLS symptoms relative to patients without augmentation and moreover faster onset of symptoms after entering a resting phase as well as spreading of symptoms to other areas of the bodies under dopaminergic therapy. An increase in intensity of symptoms is considered a further sign of augmentation, but may also be due to tolerance to the therapeutic drug. Methods to determine augmentation as side-effect of L-Dopa or dopaminergic therapies are well known in the art.

In yet another embodiment, the invention relates to a method of determining the dose of a drug used for treating a patient suffering from RLS which comprises: (a) determining if the patient carries the oligo- or polynucleotide or the presence of an SNP within the oligo- or polynucleotide of the invention as compared to the wild type sequence; (b) wherein in a patient who is tested positive in step (a) a decreased amount of said drug displaying dopaminagonistic activity is to be administered in comparison to the amount that is to be administered without regard to the patient's alleles in the MEIS1, BTBD9, MAP2K5, LBXCOR1, PTPRD or A2BP1 gene for the treatment of RLS, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

The term "alleles" refers to the specific genetic set-up of the patient with respect to the mentioned genes. This genetic set-up may or may not encompass genetic variants of said genes which comprise the oligo- or polynucleotide of the invention. As mentioned above, it is evident to the person skilled in the art that the genetic knowledge deduced from the present invention can now be used to exactly and reliably characterize the genotype of a patient. Since the prior art methods could not routinely rely on genetically manifested SNPs indicative of RLS or a predisposition therefore, due to the fact that corresponding knowledge did not exist, drugs were routinely administered in the treatment of RLS without regard to the patient's alleles of the mentioned genes. In this regard, RLS can be predicted and preventive or therapeutical measures can be applied accordingly. Moreover in accordance with the foregoing, in cases where a given drug takes an unusual effect, a suitable individual therapy can be designed based on the knowledge of the individual genetic makeup of a subject with respect to the oligo- or polynucleotides of the invention and improved therapeutics can be developed as will be further discussed below. Accordingly, by being able to reduce the dose of a currently used drug associated with side-effects in long term therapy such as, for example L-Dopa, one will prolong the therapy with said drug without said unwanted impact on the patient and cause the desired effect of alleviating symptoms in the patient. Thanks to the present invention, an improvement in therapy for RLS cases is provided with drugs already approved, thus bridging the time until more potent and less toxic medicaments are developed with methods described infra.

In a preferred embodiment of the above method said drug is selected from the group consisting of dopamine agonists, opioids, benzodiazepine receptor agonists, antiepileptic drugs or L-Dopa.

Further, the invention relates to a method of testing the efficacy of a drug used for treating RLS in treating other dopaminergic diseases, sleep disorders or spinocerebellar ataxias comprising: (a) determining if the patient or group of patients suffering from other dopaminergic diseases, sleep disorders or spinocerebellar ataxias carries the oligo- or polynucleotide of the invention or the presence of an SNP within the oligo- or polynucleotide of the invention as compared to the wild type sequence; (b) wherein in said patient or group of patients who is tested positive in step (a) said drug is to be administered and (c) assessing whether the condition of the patient or group of patients is improved after administration of said drug, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

The present invention discloses oligo- or polynucleotides or SNPs as defined above contained within said oligo- or polynucleotides that are associated with RLS. It has been demonstrated that in 80% of RLS patients also periodic limb movements (PLM, and other forms such as PLMS (periodic limb movement in sleep), PLMW (periodic limb movement while awake), a combination thereof or PLMD (periodic limb movement disorder)) occur (Montplaisir et al., Mov Disord 1997; 12:61-5). Thus, the occurrence of periodic limb movements has been established as a secondary and supportive criteria for the diagnosis of RLS. PLM is known to occur frequently in a variety of other diseases such as in sleep disorders (Montplaisir et al., Sleep Med 2000; 1:163-7), in Parkinson syndrome and other dopaminergic diseases. The current therapies for RLS with for example L-Dopa or dopamine agonists simultaneously relieve symptoms for RLS and PLM. The inventors of the present application show an attributable risk fraction (ARF) of 76.3% for the three loci combined, in other words 76.3% of RLS patients carry one of the oligo- or polynucleotides of the invention or SNPs as defined above in one of the mentioned genes. Thus, taking into consideration the simultaneous relief of symptoms of RLS and PLM in RLS patients due to RLS therapies, the high prevalence of PLM in RLS patients but also in patients with above-mentioned diseases combined with the high ARF demonstrated by the inventors, it is possible to envisage that there may be a common genetic background at least in part involving the oligo- or polynucleotides of the invention or SNPs as defined above in one of the mentioned genes in diseases such as other dopaminergic diseases, sleep disorders or spinocerebellar ataxias where periodic limb movement occurs as secondary symptom. Hence, patients or subsets of patients having the latter diseases and carrying the oligo- or polynucleotides of the invention or SNPs as defined above in one of the mentioned genes may be treated in accordance with the invention by the means and methods provided in this application. Furthermore, as drugs that have initially and exclusively been designed and used for treating dopaminergic diseases like for example Parkinson's disease have also shown a beneficial effect in RLS patients, it is now due to the present invention possible to envisage the potential beneficial effect of drugs effective in RLS patients in the treatment of dopaminergic diseases, sleep disorders or spinocerebellar ataxias. The efficacy of drugs can be determined by methods well known in the art and includes for example in vitro methods as described below or collecting data that refers to an alleviation of symptoms in said patient or group of patients.
The above applies mutatis mutandis to the method described in the following.

An embodiment of the invention relates to the use of a drug effective in treating RLS for testing the efficacy of said drug in treating other dopaminergic diseases, sleep disorders or spinocerebellar ataxias comprising: (a) determining if the patient or group of patients suffering from other dopaminergic diseases, sleep disorders or spinocerebellar ataxias carries the oligo- or polynucleotide of any one of claims 1 to 4 or the presence of an SNP within the oligo- or polynucleotide of any one of claims 1 to 4 as compared to the wild type sequence; (b) wherein in said patient or group of patients who is tested positive in step (a) said drug is to be administered and (c) assessing whether the condition of the patient or group of patients is improved after administration of said drug, wherein said SNP is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

In a preferred embodiment of the method or use of the invention said dopaminergic disease, sleep disorder or spinocerebellar ataxia is selected from the group comprising Parkinson's disease, Parkinson syndromes, doparesponsive dystonia, attention deficit hyperactivity disorder (ADHD), narcolepsy, circadian rhythm disorder, periodic limb movement disorder (PLMD) and insomnia.

Parkinson syndromes comprise diseases such as, for example multisystem atrophy, synucleinopathy or tauopathies such as, for example Alzheimer's disease.

In a further embodiment the invention relates to a method of identifying an inhibitor, pro-drug or drug capable of modulating the activity of a peptide or protein of claim 8 comprising the steps of:
(a) contacting the host cell of the invention or the peptide or protein of the invention with a molecule known to be bound by the peptide or protein of the invention to form a complex of said protein and said molecule;
(b) contacting said complex with a compound under conditions that allow maintenance of the complex between the peptide or protein of the invention and said molecule in the absence of said compound, and
(c) measuring whether said compound displaces said molecule from said complex.

The term "compound" in a method of the invention includes a single substance or a plurality of substances which may or may not be identical. Advantageously, in said method said measuring step comprises measuring the formation of a second complex of said protein and said inhibitor candidate. Preferably, said measuring step comprises measuring the amount of said first molecule that is not bound to said protein. An also preferred aspect of the above-described method is that said first molecule is an agonist or antagonist or a substrate and/or a inhibitor and/or a modulator of the polypeptide of the invention, e.g., with a radioactive or fluorescent label.

Said compound(s) may be chemically synthesized or produced via microbial fermentation but can also be comprised in, for example, samples, e.g., cell extracts from, e.g., plants, animals or microorganisms. Furthermore, said compounds may be known in the art but hitherto not known to be useful as an inhibitor, respectively. The plurality of compounds may be, e.g., added to the culture medium or injected into a cell or non-human animal of the invention. Moreover, the compound to be screened can be contained in libraries of small molecules, such as organic or inorganic small molecules which may be commercially available. In addition, libraries comprising antibodies or functional fragments or derivatives thereof (i.e. fragments or derivatives maintaining the binding specificity of the original antibody) may be used as a starting point in the screening process. Also, libraries of aptamers such as peptide aptamers might be employed. The skilled artisan is of course free to use any other starting point of desired compounds for use in the screen assays described throughout the specification. Suitable libraries are commercially available, for example from ChemBridge Corp., San Diego, USA.
If a sample containing (a) compound(s) is identified in the method of the invention, then it is either possible to isolate the compound from the original sample identified as containing the compound, in question or one can further subdivide the original sample, for example, if it consists of a plurality of different compounds, so as to reduce the number of different substances per sample and repeat the method with the subdivisions of the original sample. It can then be determined whether said sample or compound displays the desired properties, for example, by the methods described herein or in the literature (Spector et al., Cells manual; see supra). Depending on the complexity of the samples, the steps described above can be performed several times, preferably until the sample identified according to the method of the invention only comprises a limited number of or only one substance(s). Preferably said sample comprises substances of similar chemical and/or physical properties. The methods of the present invention can be easily performed and designed by the person skilled in the art, for example in accordance with other cell based assays described in the prior art or by using and modifying the methods as described herein. Furthermore, the person skilled in the art will readily recognize which further compounds may be used in order to perform the methods of the invention, for example, enzymes, if necessary, that convert a certain compound into a precursor. Such adaptation of the method of the invention is well within the skill of the person skilled in the art and can be performed without undue experimentation.

Compounds which can be used in accordance with the present invention include peptides, proteins, nucleic acids, antibodies, small organic compounds, ligands, peptidomimetics, PNAs and the like. Said compounds may act as agonists or antagonists of the invention. Said compounds can also be functional derivatives or analogues of known drugs. Methods for the preparation of chemical derivatives and analogues are well known to those skilled in the art and are described in, for example, Beilstein, Handbook of Organic Chemistry, Springer edition New York Inc., 175 Fifth Avenue, New York, N.Y. 10010 U.S.A. and Organic Synthesis, Wiley, New York, USA. Furthermore, said derivatives and analogues can be tested for their effects according to methods known in the art or as described. Furthermore, peptide mimetics and/or computer aided design of appropriate drug derivatives and analogues can be used, for example, according to the methods described below.
Appropriate computer programs can be used for the identification of interactive sites of a putative inhibitor and the peptides or proteins of the invention by computer assistant searches for complementary structural motifs (Fassina, Immunomethods 5 (1994), 114-120). Further appropriate computer systems for the computer aided design of protein and peptides are described in the prior art, for example, in Berry, Biochem. Soc. Trans. 22 (1994), 1033-1036; Wodak, Ann. N. Y. Acad. Sci. 501 (1987), 1-13; Pabo, Biochemistry 25 (1986), 5987-5991. The results obtained from the above-described computer analysis can be used in combination with the method of the invention for, e.g., optimizing known inhibitors, analogs, antagonists or agonists. Appropriate peptidomimetics and other inhibitors can also be identified by the synthesis of peptidomimetic combinatorial libraries through successive chemical modification and testing the resulting compounds, e.g., according to the methods described herein. Methods for the generation and use of peptidomimetic combinatorial libraries are described in the prior art, for example in Ostresh, Methods in Enzymology 267 (1996), 220-234 and Dorner, Bioorg. Med. Chem. 4 (1996), 709-715. Furthermore, the three-dimensional and/or crystallographic structure of said compounds and the polypeptides of the invention can be used for the design of peptidomimetic drugs (Rose, Biochemistry 35 (1996), 12933-12944; Rutenber, Bioorg. Med. Chem. 4 (1996), 1545-1558). It is very well known how to obtain said compounds, e.g. by chemical or biochemical standard techniques. Thus, also comprised by the method of the invention are means of making or producing said compounds. In summary, the present invention provides methods for identifying compounds which can be used in specific doses for the treatment of RLS.

The therapeutically useful compounds identified according to the method of the invention can be formulated and administered to a patient by methods well known in the art. Drugs or pro-drugs after their *in vivo* administration are metabolized in order to be eliminated either by excretion or by metabolism to one or more active or inactive metabolites (Meyer, J. Pharmacokinet. Biopharm. 24 (1996), 449-459). Thus, rather than using the actual compound or inhibitor identified and obtained in accordance with the method of the present invention a corresponding formulation as a pro-drug can be used which is converted into its active in the patient. Precautionary measures that may be taken for the application of pro-drugs and drugs are described in the literature; see, for review, Ozama, J. Toxicol. Sci. 21 (1996), 323-329).
The above definitions apply mutatis mutandis to the method described in the following.

In another embodiment the invention relates to a method of identifying and obtaining an inhibitor, pro-drug or drug capable of modulating the expression of a peptide or protein of the invention comprising the steps of:
(a) contacting the host cell or the peptide or protein of the invention with a molecule known to be bound by the peptide or protein of the invention to form a complex of said protein and said molecule;
(b) contacting said complex with a compound under conditions that allow maintenance of the complex between the peptide or protein of the invention and said molecule in the absence of said compound, and
(c) measuring whether said compound modulates the expression of said peptide or protein resulting to yield a higher amount or lower amount of peptide or protein compared to the amount of peptide or protein expression in the absence of said compound.

Methods to measure the expression of peptides are well known in the art and have been described herein supra.

The invention also relates to a diagnostic kit for detection of a single nucleotide polymorphism comprising the oligo- or polynucleotide, the vector, the host cell, the peptide or protein or the antibody of the invention, wherein said single nucleotide polymorphism is characterized in that it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

The kit of the invention may contain further ingredients such as selection markers and components for selective media suitable for the generation of transgenic cells and animals. The kit of the invention can be used for carrying out a method of the invention and could be, inter alia, employed in a variety of applications, e.g., in the diagnostic field or as research tool. The parts of the kit of the invention can be packaged individually in vials or other appropriate means depending on the respective ingredient or in combination in suitable containers or multicontainer units. Manufacture of the kit follows preferably standard procedures which are known to the person skilled in the art. The kit may be used for methods for detecting expression of a mutant form of the oligo- or polynucleotides, peptides or proteins in accordance with any one of the above-described methods of the invention, employing, for example, immunoassay techniques such as radioimmunoassay or enzymeimmunoassay or preferably nucleic acid hybridization and/or amplification techniques such as those described herein before as well as pharmacokinetic studies when using non-human transgenic animals.

Finally, the present invention relates to the use of wild type MEIS1, BTBD9, MAP2K5, LBXCOR1, PTPRD or A2BP1 gene for the preparation of a composition for gene therapy for the treatment or prevention of RLS.

The use of an effective dose of nucleic acid encoding a functional and expressible wild type MEIS1, BTBD9, MAP2K5, LBXCOR1, PTPRD or A2BP1 protein is intended for the preparation of a pharmaceutical composition for treating, preventing and/or delaying a disorder diagnosed by the method of the invention. A gene encoding a functional and expressible MEIS1, BTBD9, MAP2K5, LBXCOR1, PTPRD or A2BP1 protein can be introduced into cells which in turn produce the protein of interest. Gene therapy, which is based on introducing therapeutic genes into cells by *ex vivo* or *in vivo* techniques is one of the most important applications of gene transfer. Suitable vectors and methods for *in vitro* or *in vivo* gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Isner, Lancet 348 (1996), 370-374; Muhlhauser, Circ. Res. 77 (1995), 1077-1086; Wang, Nature Medicine 2 (1996), 714-716; WO 94/29469; WO 97/00957 or Schaper, Current Opinion in Biotechnology 7 (1996), 653-640, and references cited therein. The gene may be designed for direct introduction or for introduction via liposomes, or viral vectors (e.g. adenoviral, retroviral) into the cell. Preferably, said cell is a germ line cell, embryonic cell, or egg cell or derived therefrom, most preferably said cell is a stem cell.

As evident from the above, it is preferred that in the use of the invention the oligo- or polynucleotide is operatively linked to regulatory elements allowing for the expression and/or targeting of the MEIS1, BTBD9, MAP2K5, LBXCOR1, PTPRD or A2BP1 protein to specific cells. Suitable gene delivery systems that can be employed in accordance with the invention may include liposomes, receptor-mediated delivery systems, naked DNA, and viral vectors such as herpes viruses, retroviruses, adenoviruses, and adeno-associated viruses, among others. Delivery of nucleic acids to a specific site in the body for gene therapy may also be accomplished using a biolistic delivery system, such as described by Williams (Proc. Natl. Acad. Sci. USA 88 (1991), 2726-2729). Standard methods for transfecting cells are well known to those skilled in the art of molecular biology, see, e.g. WO 94/29469; see also supra. Gene therapy may be carried out by directly administering the recombinant DNA molecule or vector of the invention ex vivo and infusing cells into the patient.

The figures show:

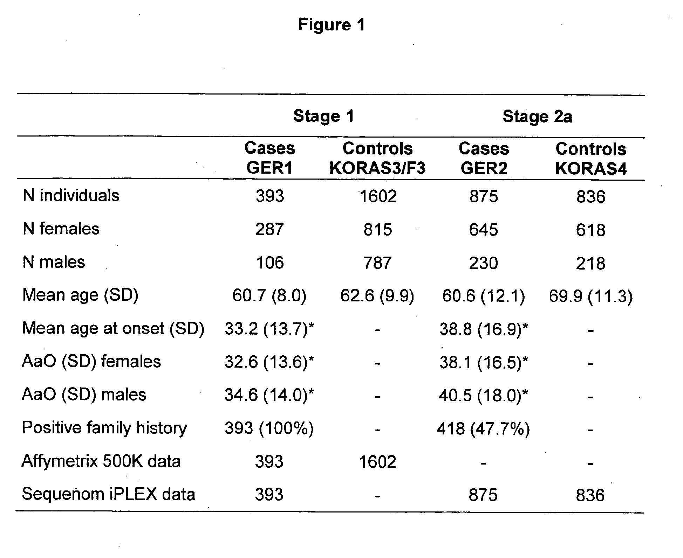

**Figure 1****. Description of study subjects**. This figure depicts a table that includes only the successfully genotyped samples stage 1 and 2a. N, number; AaO= age at onset of the disease; GER, German; KORAS3/F3 and KORAS4, controls drawn from KORA population-based cohort study, Germany. * AaO is unknown for 21 (15 females, 6 males) in GER1 and 39 cases (30 females, 9 males) in GER2.

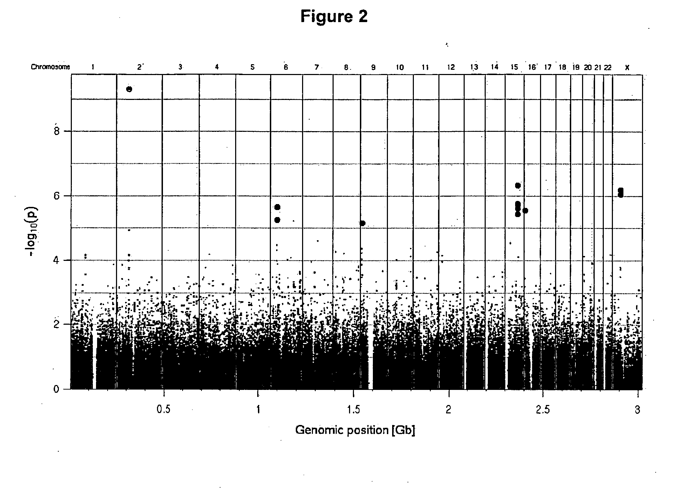

**Figure 2****. Genome-wide association study for RLS susceptibility loci**. The analysis compared 401 RLS cases with 1,644 population-based KORA controls. The x-axis is genomic position, and the y-axis is -log₁₀(P). Thirteen SNPs that passed inclusion criteria for the replication study of stage 2 are highlighted in bold. Note that the P-values of three SNPs on chromosome 15 are very similar and these SNPs appear as one single dot.
**Figure 3****. Stage 2 SNP selection.** The figure depicts a table with the 28 SNPs that were chosen for genotyping in stage 2 (13 SNPs from stage 1 and 15 additional SNPs), + indicates successfully genotyped. Genome positions (Genome pos) refer to the Human March 2006 (hg18) assembly (http://genome.ucsc.edu/index.html). * SNP, reference SNP from which the distance within the region was computed. r², r²-values between the reference *SNP and its respective neighboring SNPs as downloaded from HapMap rel21a_NCBI_Build35 (http://www.hapmap.org). ** not genotyped in stage 2 because no PCR-primer could be designed.

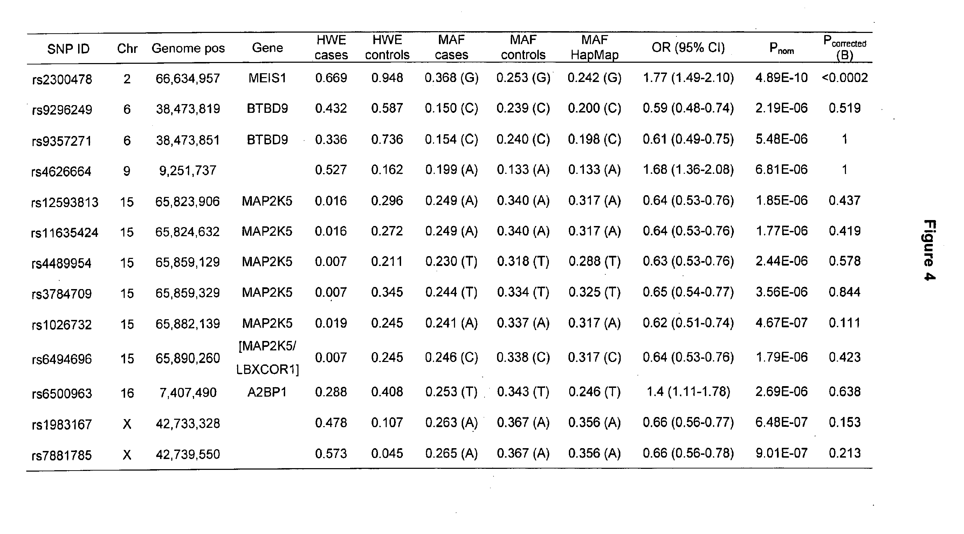

**Figure 4****. Stage 1 association results.** The figure depicts a table with 13 SNPs that showed nominally significant association and were selected for replication. Chr, Chromosome; HWE, P-value for the deviation from Hardy-Weinberg-Equilibrium; MAF, minor allele frequency; OR, odds ratio; CI, confidence interval; pₙₒₘ, nominal P-value. Genome positions (Genome pos) refer to the Human March 2006 (hg18) assembly (http://genome.ucsc.edu/index.html). [gene] denotes intergenic position of SNP. HapMap refers to HapMap rel21a_NCBI_Build35 (http://www.hapmap.org). Minor allele annotation refers to HapMap data. Nominal P-values were calculated using logistic regression implementing Armitage trend test and taking sex and age as covariates into account. Odds ratios and confidence limits were calculated from logistic regression. P-values resulting from this regression were further corrected for population stratification by dividing the resulting χ² by the inflation factor λ, ..i.e. by dividing with 1.09. P_{corrected}, P-value corrected for multiple testing using Bonferroni (B).

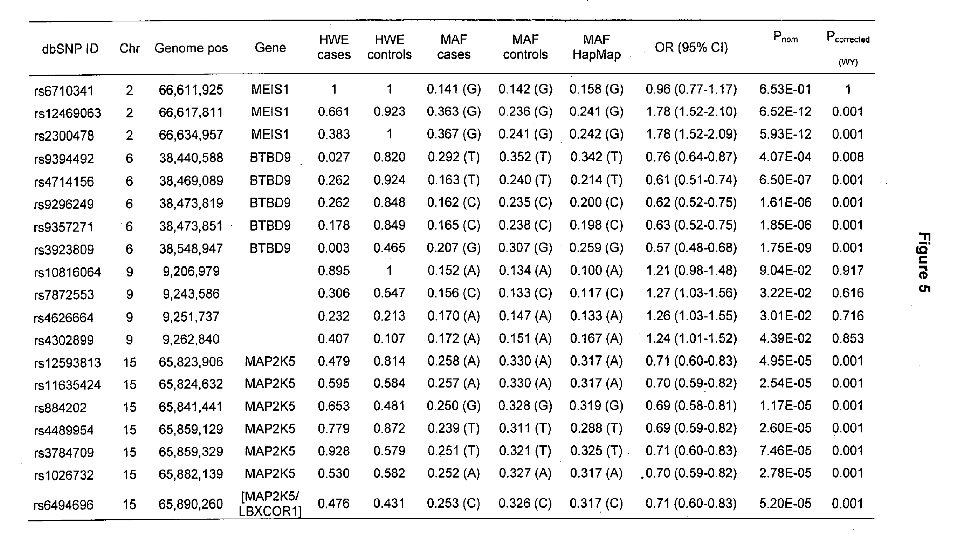

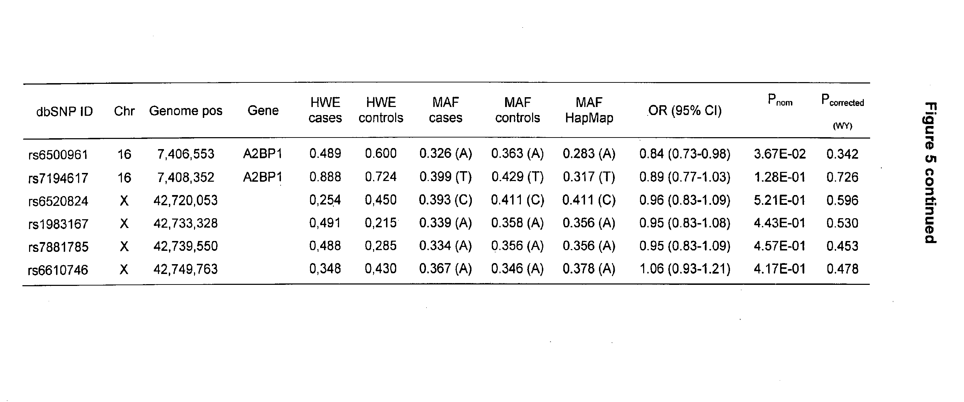

**Figure 5****. Stage 2a association results**. The figure depicts a table with SNPs that showed nominally significant association in stage 2a. Chr, Chromosome; HWE, Hardy-Weinberg-Equilibrium; MAF, minor allele frequency; OR, odds ratio; Cl, confidence interval; Pₙₒₘ, nominal P-value. Genome positions (Genome pos) refer to the Human March 2006 (hg18) assembly (http://genome.ucsc.edu/index.html). HapMap refers to HapMap re121a_NCBI_Build35 (http://www.hapmap.org). Minor allele annotation refers to HapMap data. Nominal P-values were calculated using logistic regression implementing Armitage trend test and correcting for population stratification. P_{corrected}, P-value corrected for multiple testing using Westfall-Young method (WY).
**Figure 6****. Pairwise linkage disequilibrium diagrams for three RLS associated loci.** Pairwise LD measured as D' was calculated from CEU Hapmap data. Shading represents the magnitude and significance of pairwise LD, with a white-to-red gradient reflecting lower to higher LD values. Positions of stage 2 SNPs are indicated by dotted lines. SNPs are identified by symbols whose positions show the level of significance from stage 2 genotyping on a -log₁₀(P) axis; samples are color-coded. Transcriptional units are indicated by black arrows, with exons indicated with light-grey bars. **a**, MEIS1; **b,** BTBD9; **c,** MAP2K5/LBXCOR1.

The examples illustrate the invention:

### Example 1: Study design, study population and phenotype assessment

The two-stage study design involved an exploratory stage (stage 1), followed by a replication stage in further case/control samples (stages 2a). The cases of stage 1 and 2a were recruited via specialized outpatient clinics for RLS in Munich, Marburg, Kassel, Göttingen (Germany) and Vienna (Austria) from 2000 to 2003 (sample 1) and in a second wave from 2004 to 2007 (sample 2a). In all cases diagnosis was made according to the diagnostic criteria of the International RLS Study Group assessed in a personal interview conducted by an RLS expert. A positive family history was based on the report of at least one first degree family member affected by RLS. The controls of stage 1 and 2a were recruited from the KORA S3/F3 and S4 surveys. These are representative samples from the general population living in or near the city of Augsburg, Southwest German. KORA procedures and samples have been described (Wichmann et al., Gesundheitswesen 67, S26-S30 (2005)). Consequent to informed consent, each of the surveys sampled subjects from ten strata according to sex (equal ratio) and age (range 25-74 years) with a minimum stratum size of > 400 subjects. In the KORA S3 study 4,856 subjects were studied between 1994 and 1995, and in S4 altogether 4,261 subjects between 1999 and 2001. 3006 individuals from S3 returned for follow up between 2003 and 2005 (S3/F3). For stage 1 we included 1644 subjects from S3/F3, then aged 35 to 84 years, and for stage 2 891 age and sex matched subjects from S4. 102 of cases 2a were outside the age range of KORA and were matched to the next age group (32 males, 68 females aged between 75-79 were matched with KORA 70-74; two females, both 22 years, were matched with KORA 25-29) (cf. Figure 1).

### Example 2: Genomewide assay, genotyping and quality control

Stage 1 genotyping was performed using the Affymetrix 500K Array Set. Hybridization of genomic DNA was performed in accordance with the manufacturer's standard recommendations (Affymetrix Inc,. *http:*//*www.affymetrix. com*/*support*/*technical*/*whitepapers*/*brlmm_whitepaper.pdf* (2006)). Genotypes were determined using BRLMM clustering algorithm. All samples of stage 1 underwent a joint BRLMM cluster analysis.
From a total of 500,568 SNPs from the Affymetrix chip, 4078 were monomorph. From the other 496,490 SNPS the following number of SNPs were discarded from subsequent analyses for the following reasons: low call rate (< 98 %, n = 148,342), low heterozygosity (< 10 heterozygotes, n = 34,215), low minor allele frequency (MAF < 0.10, n = 158,953), and significant deviations from HWE (P< 0.00001 in controls) (n = 31,160). Thus a total of 236,758 SNPs could be used for the subsequent analyses. By genotyping 400 samples with 15 SNPs in a second replicate we calculated a genotype discordance rate of 0.2 %. Thirteen SNPs passed the inclusion criteris for stage 2 (cf. Figure 3).

Stage 2 genotyping was performed using matrix-assisted laser desorption/ionization time-of-flight (MALDI-TOF) mass spectrometry on a Sequenom MassArray system (Autoflex HT, Sequenom). Cleaned extension products were analysed by a mass spectrometer (Bruker Daltronik), and peaks were identified using the SpectroTYPER RT 3.4 software (Sequenom). Assays were designed by the AssayDesign software 3.1.2.2 (Sequenom) with the default parameters for the iPLEX Gold chemistry, unbiased assay quality was assessed with no knowledge of subject phenotype before the results were subjected to statistical analysis. SNP rs6500693 from stage 1 was not genotyped in stage 2 because no PCR-primers could be designed. In each sample four SNPs had a callrate < 95% and were disregarded from all further analysis. In total 30 SNPs were successfully genotyped in stage 2a.
A total of 32 SNPs were genotyped. Two SNPs (rs6747972, rs2110974) in stage 2a were excluded due to a low call rate (< 95%).

SNP Selection for stage 2. For the inclusion of loci into stage 2a, the following criteria were applied:
1) A P-value of less than 10⁻⁶ in the stage 1 analysis led to an automatic inclusion of any SNP into the follow up. This led to the inclusion of 4 SNPs.
2) Every SNP with a P-value equal to or below 10⁻⁵ with two neigbouring SNPs (+/-100kb) with a P ≤ 10⁻³, leading to the inclusion of 8 SNPs.
3) Within described linkage peaks, every SNP with a P- value equal to or below 10⁻⁴ was included. This amounted to one additional SNP.
For the SNPs passing either criterion 1, 2, or 3, visual inspection of clustering data was performed using Affymetrix' SNP Signaling Tool v 1.0.2. In all cases clusters were of good quality, thus none of the included 13 SNPs could be excluded on these grounds. These 13 SNPs defined six discernible regions. An additional 15 SNPs contained within these regions were chosen for genotyping in an (stage 2a) independent population based on linkage disequilibrium (LD) structure: We choose one of the original SNPs for each of the seven regions and sought one SNP with an r²-value of ≥ 0.9 as a technical replicate, two SNPs with an r² ≈ 0.8 and two SNPs with an r² ≈ 0.7 upstream and downstream, respectively, of the original SNP. In regions where these criteria could not be met, neighbouring SNPs with maximum r² obtainable in this region were chosen (cf. Figure 3).
For the stage 2 replication, only 13 SNPs (cf. Figure 4) passed our inclusion criteria based on P-values, putative location within a linkage peak, and visual inspection of clustering data. These 13 SNPs, mapping to six discernible regions plus 15 neighbouring SNPs were selected for replication. Of these, 12 original SNPs and 13 additional SNPs were successfully genotyped in stage 2a, and 11 original SNPs and 13 additional SNPs were successfully genotyped in stage 2b (cf. Figure 3). Cases of stage 2a were recruited in a second recruitment wave using the same sampling design as in stage 1. The case sample included 903 RLS subjects with familial and sporadic RLS. Control subjects were selected from KORA S4 (n = 891). A total of 28 cases and 55 controls were excluded due to low mean call rates (< 90 %).
In stage 2a we found nominally significant evidence for association in six regions (cf. Figure 5). Three of these regions withstood correction for multiple testing.

### Example 3: Statistical analysis of genetic effects

To test and correct for possible population stratification, we performed an EIGENSOFT (Price et al., Nat. Genet. 38, 904-909 (2006); Patterson et al., PLos Genet. 2, e190 (2006)) analysis of case and control genotypes for all stages.
In stage 1 we performed logistic regression analysis coding the number of minor alleles at the SNPs as the dependent variables, thus implementing Armitage's test of trend, including age and sex as covariates as well as allowing for interactions between age, sex, and the number of alleles. Odds ratios and confidence interval limits thereof were obtained through this logistic regression analysis. In stage 2a, we performed the same analysis. Because stage 2a was matched for age and sex, these factors were not included as covariates. Chi-square values resulting from these analyses were divided by the value of λ found, assuming that for both German samples similar conditions should apply. Exploratory re-analysis for stage 2a including sex and age yielded results virtually identical with those not incorporating sex and age.
Multiple testing. Using the WG-PERMER tool, preliminary analysis applying the Westfall-Young min-p correction showed that, probably due to our strict criteria for SNPs to enter the analysis and resulting low average r² values between SNPs, the corrected P-values for the Westfall-Young correction and a Bonferroni correction with the number of tests set at the number of SNPs tested (n=236,758) were in very good agreement. Thus to maintain comparability across results reported, we report Bonferroni-corrected P-value using for the results of stage 1 and for the combined analysis. For stage 2a we give Westfall-Young corrected P-values as here.
Power analysis for the combined German sample was performed using the Genetic Power Calculator (http://pngu.mgh.harvard.edu/~purcell/gpc/). Any allele frequency of 0.2 or more and an odds ratio of 1.5 or large (or 1/1.5 or lower) was beyond 90% in all cases.

## Claims

1. An oligo- or polynucleotide comprising or consisting of an oligo- or polynucleotide selected from the group consisting of:
(a) an oligo- or polynucleotide consisting of at least 17 consecutive nucleotides of any of SEQ ID NOs: 1 to 40; wherein the at least 17 consecutive nucleotides contain a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40;
(b) an oligo- or polynucleotide hybridizing under stringent conditions to at least a portion of the oligo- or polynucleotide of (a), wherein said portion comprises the nucleotide in position 101 of any of SEQ ID NOs: 1 to 40, wherein said oligo- or polynucleotide contains a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40;
(c) an oligo- or polynucleotide identical to the oligo- or polynucleotide of (a) or (b) with the exception that T is replaced by U.

2. The oligo- or polynucleotide of claim 1 which is DNA.

3. The oligo- or polynucleotide of claim 1 or 2 which is a gene.

4. The oligo- or polynucleotide of claim 3 wherein the gene is MEIS1, BTBD9, MAP2K5, LBXCOR1, PTPRD or A2BP1.

5. A vector comprising an oligo- or polynucleotide of any one of claims 1 to 4.

6. A host cell genetically engineered with the oligo- or polynucleotide of any one of claims 1 to 4 or the vector of claim 5.

7. A method for the production of a peptide or protein encoded by the oligo- or polynucleotide of any one of claims 1 to 4 comprising culturing the host of claim 6 under conditions allowing the expression of the peptide or protein and recovering the peptide or protein.

8. A peptide or protein encoded by the oligo- or polynucleotide of any one of claims 1 to 4 or the vector of claim 5 or obtainable by the method of claim 7.

9. An antibody which binds specifically to the peptide or protein of claim 8.

10. A method of diagnosing RLS or a predisposition for RLS comprising determining (a) the presence of an oligo- or polynucleotide of any one of claims 1 to 4 or (b) the presence of an SNP within the oligo- or polynucleotide of any one of claims 1 to 4 as compared to the wild type sequence in a sample from a subject, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40 and wherein the presence of said oligo- or polynucleotide or said SNP is indicative of RLS or a predisposition for RLS.

11. The method of claim 10 further comprising determining the presence of a peptide or protein of claim 8 or an amino acid exchange within the peptide or protein that is a consequence of an SNP as defined in claim 10 within the oligo-or polynucleotide of any one of claims 1 to 4 as compared to the wild type sequence.

12. A method of diagnosing RLS or a predisposition for RLS comprising determining (a) the presence of a peptide or protein of claim 8 or (b) the presence of an SNP within the oligo- or polynucleotide of any one of claims 1 to 4 as compared to the wild type sequence in a sample from a subject, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40 and wherein the presence of said peptide or protein or said SNP is indicative of RLS or a predisposition for RLS.

13. The method of any one of claims 10 to 12 wherein said determining of the presence of said peptide or protein or the presence of said SNP comprises PCR based techniques, RFLP-based techniques, DNA sequencing-based techniques, hybridization techniques, single-strand conformation polymorphism analysis (SSCA), denaturating gradient gel electrophoresis (DGGE), mismatch cleavage detection, heteroduplex analysis, techniques based on mass spectroscopy, HPLC-based techniques, primer extension-based techniques, and 5'-nuclease assay-based techniques.

14. The method of any one of claims 10 to 12, further comprising detecting an alteration in the expression of the peptide or protein of claim 8 or wild type MEIS1, BTBD9, MAP2K5, LBXCOR1, PTPRD or A2BP1 protein, wherein the alteration in expression results in either a higher amount or lower amount of peptide or protein compared to the amount of peptide or protein expression in the control sample, wherein the alteration of expression is indicative RLS or of a predisposition for RLS.

15. A method of diagnosing RLS or a predisposition for RLS comprising detecting an alteration in the expression of the peptide or protein of claim 8 or wild type MEIS1, BTBD9, MAP2K5, LBXCOR1, PTPRD or A2BP1 protein, wherein the alteration in expression results in either a higher amount or lower amount of peptide or protein compared to the amount of peptide or protein expression in the control sample, wherein the alteration of expression is indicative of RLS or a predisposition for RLS.

16. The method of claim 14 or 15, wherein the alteration in expression is detected on the basis of levels of mRNA encoding the peptide or protein of claim 8 or wild type MEIS1, BTBD9, MAP2K5, LBXCOR1, PTPRD orA2BP1 protein.

17. A method for selecting a therapy to prevent and/or treat RLS, wherein said method comprises:
(a) determining the presence or absence of an oligo- or polynucleotide of any one of claims 1 to 4 or the presence or absence of an SNP within the oligo- or polynucleotide of any one of claims 1 to 4 as compared to the wild type sequence in a sample obtained from a subject, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position, 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40; and
(b) selecting a therapy for said subject based on the results obtained in (a).

18. The method of claim 17 wherein after step (a) and prior to step (b) in step (a') the ability to develop augmentation under L-Dopa or dopamine agonist therapy is determined; and wherein step (b) comprises selecting a therapy for said subject based on the results obtained in (a) and (a').

19. A method of determining the dose of a drug used for treating a patient suffering from RLS which comprises: (a) determining if the patient carries the oligo- or polynucleotide of any one of claims 1 to 4 or the presence of an SNP within the oligo- or polynucleotide of any one of claims 1 to 4 as compared to the wild type sequence; (b) wherein in a patient who is tested positive in step (a) a decreased amount of said drug displaying dopaminagonistic activity is to be administered in comparison to the amount that is to be administered without regard to the patient's alleles in the MEIS1, BTBD9, MAP2K5, LBXCOR1, PTPRD or A2BP1 gene for the treatment of RLS, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

20. The method of claim 18 wherein said drug is selected from the group consisting of dopamine agonists, opioids, benzodiazepine receptor agonists, antiepileptic drugs or L-Dopa.

21. A method of testing the efficacy of a drug used for treating RLS in treating other dopaminergic diseases, sleep disorders or spinocerebellar ataxias comprising: (a) determining if the patient or group of patients suffering from other dopaminergic diseases, sleep disorders or spinocerebellar ataxias carries the oligo- or polynucleotide of any one of claims 1 to 4 or the presence of an SNP within the oligo- or polynucleotide of any one of claims 1 to 4 as compared to the wild type sequence; (b) wherein in said patient or group of patients who is tested positive in step (a) said drug is to be administered and (c) assessing whether the condition of the patient or group of patients is improved after administration of said drug, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

22. Use of a drug effective in treating RLS for testing the efficacy of said drug in treating other dopaminergic diseases, sleep disorders or spinocerebellar ataxias comprising: (a) determining if the patient or group of patients suffering from other dopaminergic diseases, sleep disorders or spinocerebellar ataxias carries the oligo- or polynucleotide of any one of claims 1 to 4 or the presence of an SNP within the oligo- or polynucleotide of any one of claims 1 to 4 as compared to the wild type sequence; (b) wherein in said patient or group of patients who is tested positive in step (a) said drug is to be administered and (c) assessing whether the condition of the patient or group of patients is improved after administration of said drug, wherein said SNP is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ LD NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

23. The method of claim 21 or the use of claim 22 wherein said dopaminergic disease, sleep disorder or spinocerebellar ataxia is selected from the group comprising Parkinson's disease, Parkinson syndromes, doparesponsive dystonia, attention deficit hyperactivity disorder (ADHD), narcolepsy, circadian rhythm disorder, periodic limb movement disorder (PLMD) and insomnia.

24. A method of identifying an inhibitor, pro-drug or drug capable of modulating the activity of a peptide or protein of claim 8 comprising the steps of:
(a) contacting the host cell of claim 6 or the peptide or protein of claim 8 with a molecule known to be bound by the peptide or protein of claim 8 to form a complex of said peptide or protein and said molecule;
(b) contacting said complex with a compound under conditions that allow maintenance of the complex between the peptide or protein of claim 8 and said molecule in the absence of said compound, and
(c) measuring whether said compound displaces said molecule from said complex.

25. A method of identifying and obtaining an inhibitor, pro-drug or drug capable of modulating the expression of a peptide or protein of claim 8 comprising the steps of:
(a) contacting the host cell of claim 6 or the peptide or protein of claim 8 with a molecule known to be bound by the peptide or protein of claim 8 to form a complex of said peptide or protein and said molecule;
(b) contacting said complex with a compound under conditions that allow maintenance of the complex between the peptide or protein of claim 8 and said molecule in the absence of said compound, and
(c) measuring whether said compound modulates the expression of said peptide or protein resulting to yield a higher amount or lower amount of peptide or protein compared to the amount of peptide or protein expression in the absence of said compound.

26. A diagnostic kit for detection of a single nucleotide polymorphism comprising the oligo- or polynucleotide of any one of claims 1 to 3, the vector of claim 4, the host cell of claim 5, the peptide or protein of claim 7, or the antibody of claim 8, wherein said single nucleotide polymorphism is **characterized in that** it is a G at position 101 of SEQ ID NO: 1, a C at position 101 of SEQ ID NO: 2, a G at position 101 of SEQ ID NO: 3, a C at position 101 of SEQ ID NO: 4, a C at position 101 of SEQ ID NO: 5, a G at position 101 of SEQ ID NO: 6, a C at position 101 of SEQ ID NO: 7, a G at position 101 of SEQ ID NO: 8, a T at position 101 of SEQ ID NO: 9, an A at position 101 of SEQ ID NO: 10, a T at position 101 of SEQ ID NO: 11, an A at position 101 of SEQ ID NO: 12, an A at position 101 of SEQ ID NO: 13, a T at position 101 of SEQ ID NO: 14, a G at position 101 of SEQ ID NO: 15, a C at position 101 of SEQ ID NO: 16, a G at position 101 of SEQ ID NO: 17, a C at position 101 of SEQ ID NO: 18, an A at position 101 of SEQ ID NO: 19, a T at position 101 of SEQ ID NO: 20, a G at position 101 of SEQ ID NO: 21, a C at position 101 of SEQ ID NO: 22, a C at position 101 of SEQ ID NO: 23, a G at position 101 of SEQ ID NO: 24, a G at position 101 of SEQ ID NO: 25, a C at position 101 of SEQ ID NO: 26, a G at position 101 of SEQ ID NO: 27, a C at position 101 of SEQ ID NO: 28, an A at position 101 of SEQ ID NO: 29, a T at position 101 of SEQ ID NO: 30; a C at position 101 of SEQ ID NO: 31, a G at position 101 of SEQ ID NO: 32, an A at position 101 of SEQ ID NO: 33, a T at position 101 of SEQ ID NO: 34, an A at position 101 of SEQ ID NO: 35, a T at position 101 of SEQ ID NO: 36, a G at position 101 of SEQ ID NO: 37, a C at position 101 of SEQ ID NO: 38, a T at position 101 of SEQ ID NO: 39 or an A at position 101 of SEQ ID NO: 40.

27. Use of wild type MEIS1, BTBD9, MAP2K5, LBXCOR1, PTPRD or A2BP1 gene for the preparation of a composition for gene therapy for the treatment or prevention of RLS.
